# EUROPEAN PATENT SPECIFICATION

(11) **EP 1 776 456 B1**
(45) Date of publication and mention of the grant of the patent: **20.02.2013**
(21) Application number: 05760780.6
(22) Date of filing: 03.06.2005
(51) Int. Cl.: C12Q 1/68, C12N 9/00, C12N 9/06, C12N 1/20, A61K 39/12, A61K 39/00, A61K 39/02, A61K 35/00, A61K 39/385, A61K 38/00, A01N 43/04, C07H 21/04, C12N 9/10

(54) **ISOLATED CHIMERIC PROTEINS OF MODIFIED LUMAZINE SYNTHASE**
ISOLIERTE CHIMÄRISCHE PROTEINE VON MODIFIZIERTER LUMAZIN-SYNTHASE
PROTEINES CHIMERES ISOLEES DE LUMAZINE SYNTHASE MODIFIEE

(30) Priority: 03.06.2004 AR 0401923
(43) Date of publication of application: 25.04.2007
(73) Proprietor: Goldgene LLC, 1405 Buenos Aires (AR)
(72) Inventor: GOLDBAUM, Fernando, A., 1405 Buenos Aires (AR); AINCIART, Natalia, Buenos Aires (AR); CASSATARO, Juliana, Buenos Aires (AR); FOSSATI, Carlos Alberto, La Platta (AR); VELIKOVSKY, Carlos A., Buenos Aires (AR); GIAMBARTOLOMEI, Guillermo, Buenos Aires (AR); LAPLAGNE, Diego Andrés, Buenos Aires (AR); CRAIG, Patricio, Buenos Aires (AR); ZYLBERMAN, Vanesa, Buenos Aires (AR); BERGUER, Paula Mercedes, Buenos Aires (AR)
(74) Representative: Zardi, Marco
(86) International application number: PCT/US2005/019289
(87) International publication number: WO 2005/121330

(56) References cited:
- WO-A-00/53229
- VIZCAINO NIEVES ET AL: "Minor nucleotide substitutions in the omp31 gene of Brucella ovis result in antigenic differences in the major outer membrane protein that it encodes compared to those of the other Brucella species" INFECTION AND IMMUNITY, vol. 69, no. 11, November 2001 (2001-11), pages 7020-7028, XP002478634 ISSN: 0019-9567
- GOLDBAUM FERNANDO A ET AL: "Characterization of an 18-kilodalton Brucella cytoplasmic protein which appears to be a serological marker of active infection of both human and bovine brucellosis" JOURNAL OF CLINICAL MICROBIOLOGY, vol. 31, no. 8, 1993, pages 2141-2145, XP002478635 ISSN: 0095-1137
- BALDI P C ET AL: "Structural, functional and immunological studies on a polymeric bacterial protein" BRAZILIAN JOURNAL OF MEDICAL AND BIOLOGICAL RESEARCH, vol. 33, no. 7, July 2000 (2000-07), pages 741-747, XP002478636 ISSN: 0100-879X
- VELIKOVSKY CARLOS A ET AL: "Brucella Lumazine synthase elicits a mixed Th1-Th2 immune response and reduces infection in mice challenged with Brucella abortus 544 independently of the adjuvant formulation used." INFECTION AND IMMUNITY, vol. 71, no. 10, October 2003 (2003-10), pages 5750-5755, XP002478637 ISSN: 0019-9567
- TOLEDO ANDREA ET AL: "Two epitopes shared by Taenia crassiceps and Taenia solium confer protection against murine T. crassiceps cysticercosis along with a prominent T1 response" INFECTION AND IMMUNITY, vol. 69, no. 3, March 2001 (2001-03), pages 1766-1773, XP002478638 ISSN: 0019-9567
- DUCHAINE T ET AL: "A novel murine Staufen isoform modulates the RNA content of Staufen complexes" MOLECULAR AND CELLULAR BIOLOGY, AMERICAN SOCIETY FOR MICROBIOLOGY, WASHINGTON, US, vol. 20, no. 15, August 2000 (2000-08), pages 5592-5601, XP002978700 ISSN: 0270-7306
- VELIKOVSKY CARLOS A ET AL: "A DNA vaccine encoding lumazine synthase from Brucella abortus induces protective immunity in BALB/c mice" INFECTION AND IMMUNITY, vol. 70, no. 5, May 2002 (2002-05), pages 2507-2511, XP002478639 ISSN: 0019-9567
- ZYLBERMAN VANESA ET AL: "High order quaternary arrangement confers increased structural. stability to Brucella sp. lumazine synthase." JOURNAL OF BIOLOGICAL CHEMISTRY, vol. 279, no. 9, 27 February 2004 (2004-02-27), pages 8093-8101, XP002478640 ISSN: 0021-9258
- LAPLAGNE D A ET AL: "Engineering of a polymeric bacterial protein as a scaffold for the multiple display of peptides" PROTEINS STRUCTURE FUNCTION AND BIOINFORMATICS, vol. 57, no. 4, 18 August 2004 (2004-08-18), pages 820-828, XP002478641
- SCIUTTO E ET AL: "Brucella spp. lumazine synthase: a novel antigen delivery system" VACCINE, BUTTERWORTH SCIENTIFIC. GUILDFORD, GB, vol. 23, no. 21, 15 April 2005 (2005-04-15), pages 2784-2790, XP004797048 ISSN: 0264-410X
- ROSAS ET AL: "Brucella spp. lumazine synthase: a novel adjuvant and antigen delivery system to effectively induce oral immunity" MICROBES AND INFECTION, ELSEVIER, PARIS, FR, vol. 8, no. 5, April 2006 (2006-04), pages 1277-1286, XP005489443 ISSN: 1286-4579
- CASSATARO ET AL: "A recombinant subunit vaccine based on the insertion of 27 amino acids from Omp31 to the N-terminus of BLS induced a similar degree of protection against B. ovis than Rev.1 vaccination" VACCINE, BUTTERWORTH SCIENTIFIC. GUILDFORD, GB, vol. 25, no. 22, 6 May 2007 (2007-05-06), pages 4437-4446, XP022062922 ISSN: 0264-410X
- CRAIG P O ET AL: "Multiple display of a protein domain on a bacterial polymeric scaffold" PROTEINS STRUCTURE FUNCTION AND BIOINFORMATICS, vol. 61, no. 4, December 2005 (2005-12), pages 1089-1100, XP002478642 ISSN: 0887-3585
- SCIUTTO E. ET AL.: 'Iumazinesynthase: a novel antigen delivery system' VACCINE vol. 23, no. 21, 15 April 2005, pages 2784 - 2790, XP004797048
- LAPLAGNE D. ET AL.: 'Engineering of a polymeric bacterial protein as a scaffold for the multiple display of peptides' PROTEINS vol. 57, no. 4, 01 December 2004, pages 820 - 828, XP002478641
- MAHAIRAS ET AL.: 'Molecular analysis of genetic differences between Mycobacterium bovis BCG and virulent M. bovis' J. BACTERIOL. vol. 178, no. 5, March 1996, pages 1274 - 1282, XP000647583

## Description

### Technical Description of the Invention

Isolated chimeric proteins including up to ten copies of peptides, polypeptides or protein domains inserted in the amino termini of the *Brucella spp,* lumazine synthase enzyme. Isolated nucleotides sequences codifying the chimeric proteins. Vectors, plasmids and transformed cells used for expressing the proteins. Monoclonal and polyclonal antibodies induced by the chimeric proteins. Hybridomas producing monoclonal antibodies. Vaccines and pharmaceutical compounds including the chimeric proteins, nucleotide sequences and antibodies. A method to induce an immune response in higher organisms including the administration of effective amounts of the vaccines and pharmaceutical compounds. Biosensors including the chimeric proteins. Protein conjugates formed by the chimeric proteins and a ligand bound by means of covalent and noncovalent bonds, Uses of the chimeric proteins, nucleotide sequences, vectors, plasmids, transformed cells, antibodies, hybridomas, conjugates, biosensors, vaccines and pharmaceutical compounds. The quaternary structure of the chimeric proteins.

### Technical Field of the Invention

The present invention relates to chimeric proteins formed by modified proteins derived from the lumazine synthase enzyme of *Brucella spp.,* linked to peptides, polypeptides or proteins domains. The chimeric proteins are useful for inducing immune responses in higher animals and for other purposes. The present invention relates also to pharmaceutical compositions including antigens or antibodies, or segments of antigens or antibodies, bound to the modified proteins.

### Background of the Invention

The massive application of live-attenuated vaccines offer several economic and health inconveniences. For example, when live vaccines are attenuated their immunogenic capability is often reduced. *See* Leclerc, et al., Immunol. Today, 19(7):300-302, (1998); Nieba, et al., Mod Asp. Immunobiol., 1(2):36-39 (2000). Another inconvenience is the possibility that the attenuation be reverted and that the microorganism regain its disease inducing properties. *See* Redfield, N., N. Eng. J. Med., 316:673-678 (1998). Hence, during the past years, the trend has been to formulate acellular vaccines, based on individual compounds isolated from bacteria or virus. In general, these individual compounds, like proteins typical of a microorganism, have low immunogenicity. This limitation has been overcome by using adjuvant substances. However, there are proteins that even in the presence of adjuvant substances continue showing low immunogenicity. Several protein engineering strategies have been proposed to overcome these difficulties. *See* Leclerc, et al., Immunol. Today, 19(7):300-302 (1998).

Viral capsid proteins are able to form tridimensionally ordered particles, called "virus-like particles". These particles have the same size and shape as whole viruses. However, they are empty inside and without genetic material rendering them incapable of producing infections. Their great size and order provide them with a marked immunogenicity. *See* Bachmann, et al., Science, 262:1448-1451 (1993). The recombinant vaccine against hepatitis B, widely accepted in the market, is based on this concept. The "virus-like particles" have been used as a vehicle for inserting peptides characteristic of certain pathogens with the aim of producing vaccines against such pathogenic microorganisms. *See* WO0032227 (Renner, et al.); W00185208 (Bachmann, et al.). A favored strategy has been the insertion of multiple copies of a peptide in a very immunogenic vehicle in order to provide the peptide with the adjuvant property of the carrier. However, this approach has encountered many difficulties: owing to the huge size of these particles, any insertion of a peptide in its compounding protein obstructs its proper folding and, in many cases, decreases its stability. Moreover, there are few protein sites able to accept peptide insertions without changing their general structure. *See* Nieba, et al. Mod. Asp. Immunobiol., 1(2):36-39 (2000).

Some bacterial proteins have been postulated as vehicles for developing chimeric vaccines. *See* Leclerc, et al., Immunol Today, 19(7):300-302 (1998). The subunit B of the cholera toxin is a stable pentameric protein that has been used to obtain an immune response from the mucosa against inserted peptides. This strategy has been successful due to this toxin capacity to penetrate the gastric mucosa. *See* Arakawa, et al. Nature Biotech, 16:934-938 (1998). The dihydrolipoyl dehydrogenase enzyme of the *Bacillus steearothermophilus* has also been postulated as a proteic vehicle because it forms a complex and very stable polymeric structure. *See* Domingo, et al., J. Mol. Biol., 305:259-267 (2001); WO0142439 (Domingo, et al.).

The lumazine synthase enzyme catalyzes the penultimate step in the riboflavin biosynthetic route. *See* Goldbaum, et al., J. Med Microbiol., 48: 833-839 (1999). Its active site is located in the interphase among monomers, making this protein to have a very stable polymeric order. *See* Ritsert, et al. J. Mol. Biol., 253:151-167 (1995). These orders vary between proteins forming pentameric and icosahedric particles. *See* Braden, et al., J. Mol. Biol., 297:1031-1036 (2000). The icosahedric structure of the lumazine synthase of *B. subtilis* has been postulated as a vehicle for inserting peptides and developing vaccines. *See* WO005322 (Bacher, et al.).

The lumazine synthase of *Brucella spp.* is a highly stable protein. It has been demonstrated that this 18-kDa protein is a useful marker for the serological diagnosis of human and animal brucellosis. *See* Goldbaum, et al., J. Clin. Microbiol., 30:604-607 (1992); Goldbaum, et al., J. Clin. Microbiol., 31:2141-2145 (1993); Baldi, et al., Clin. Diag. Lab. Immunol., 3 (4):472-476 (1996), According to immunochemical, enzymatic function and tridimensional structure by X-ray crystallography analyses the original unmodified protein shows the same folding when expressed recombinantly as the native protein. *See* Braden, et al. J. Mol. Biol, 297:1031-1036 (2000); Goldbaum, et al., J. Med. Microbiol., 48:833-839 (1999); Goldbaum, et al., J. Struct. Biol., 123:175-178 (1998). The structure shows that this 18-kDa protein behaves as a 180-kDa decamer in solution, becoming a new type of quaternary arrangement of the lumazine synthase. *See* Zylberman, et al., J. Biol. Chem., 279(9):8093-8101 (2004),

It has been postulated that the immunogenicity of the lumazine synthase of *Brucella spp.* derives mainly from its polymeric character. *See* Baldi, et al., Braz. J. Med. Biol. Res., 33:741-747 (2000). The structure also shows that the amino termini end of 10 amino acids is involved neither in the general folding nor in the contacts among monomers. *See* Braden, et al. J. Mol. Biol., 297:1031-1036 (2000). The lumazine synthase of *Brucella spp.* is a powerful immunogen capable of producing a high humoral and cellular immune response in the murine model. This capability has been verified when the immunization is induced with the recombinant unmodified protein and with a plasmid that codifies for the protein (gene therapy, DNA vaccination). *See* Velikovsky, et al., J. Immunol. Meth, 244(1-2):1-7 (2000). It is possible to modulate the response by changing the immunization route and the adjuvant used. *See* Velikovsky, et al., Infec. Immun. 70(5):2507-11 (2002). Particularly, it is possible to create a strong response of the T_{H}1 type, which would be the response with the highest protecting capacity in brucellosis. *See* Velikovsky, et al. Infec. Immun, 70(5):2507-11 (2002).

Velikowsky et al., Infection and Immunity, 71(10), Oct.2003, p. 5750-5755 describes the immunogenic and protective efficacy of recombinant lumazine synthase from Brucella ssp. in mice.

Toledo et al. Infection and Immunity, 69(3), Mar.2001, p.1766-1773 describes recombinant antigens KETc1 and KETc12 shared by Taenia crassiceps and Taenia solium, enhancing immunoprotection against experimental T.crassiceps cysticercosis.

Duchaine et al. (Molecular and Cellular Biology, American Society for microbiology, Washington US, vol.20(15), Aug.2000, p.5592-5601 describes human and murine taufen-1 proteins and their structural characterization, domain structure, including the RBD3 domain.

WO 00/53229 describes immunogenic fusion proteins comprising lumazine synthase from various microorganisms including *inter alia* Brucella abortus.

Zylberman et al., J.Biol.Chem., 2004, 279(9), p.8093-8101 describes the decameric quaternary arrangement of *Brucella* sp. lumazine synthase. However, there is still a need in the art for new vehicles with smaller stable structures useful for displaying peptides, polypeptides and proteins, in general, and antigens or immune response-inducing agents, in particular. The development and use of the lumazine synthase decameric structure as a vector of this type have not been described in the art

### Deposit of Microorganisms

The pBLS-OMP31 plasmid was deposited on April 1^{st}, 2004, under access number DSM 15546 in DSMZ - Deutsche Sammlung von Mikroorganismen und Zellkulturen, Mascheroder Weg 1B, D-38124 Braunschweig, Germany.

### Diagrams

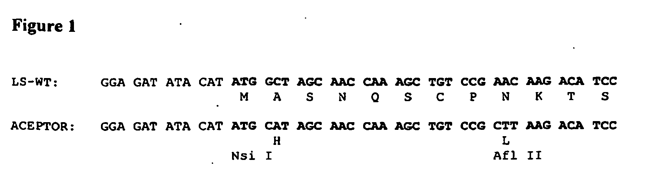

Figure 1 shows the nucleotide and amino acid sequence of the 5' end of the gene of the cloned BLS gene in the expression vector pET11a (Novagen, USA) and the generated cassette. The section in bold case shows the codifying region. The section in red case shows the mutated bases in the cassette. The section highlighted in yellow shows the new restriction sites.

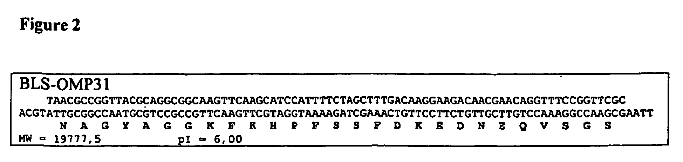

Figure 2 shows the oligonucleotides used to generate the BLS-OMP31 chimera. Their annealing produces the appearance of cohesive sites corresponding to the NsiI (5' end) and AflII (3' end) restriction enzymes, the sequence of 27 aminoacids inserted in the chimera (below, in bold case) being located among them. The theoretical molecular weight of the resulting protein monomer (19,977.5 Daltons) and the theoretical isoelectric point (p1=6.00) of the inserted peptide are also shown.

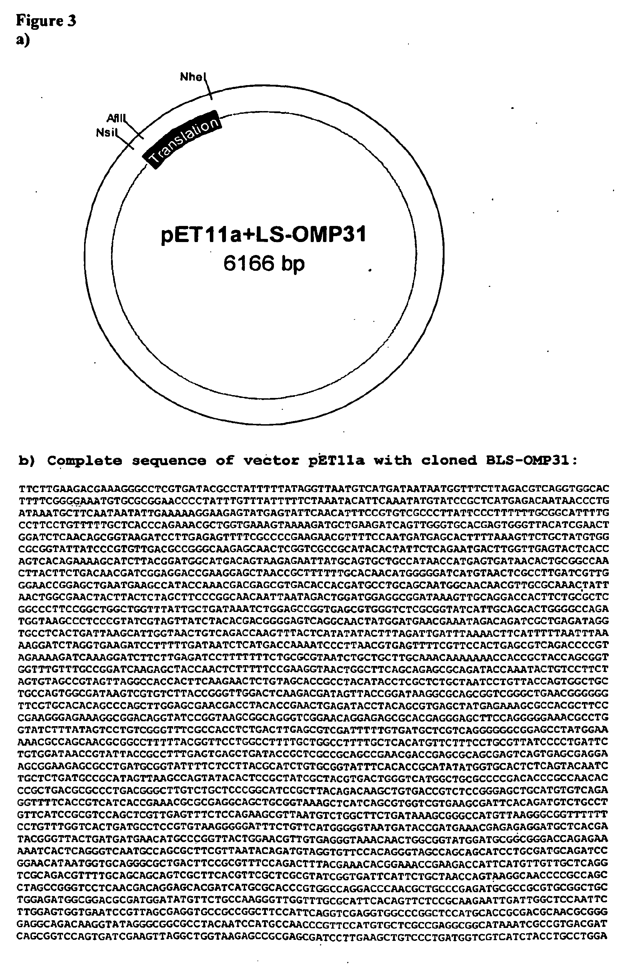

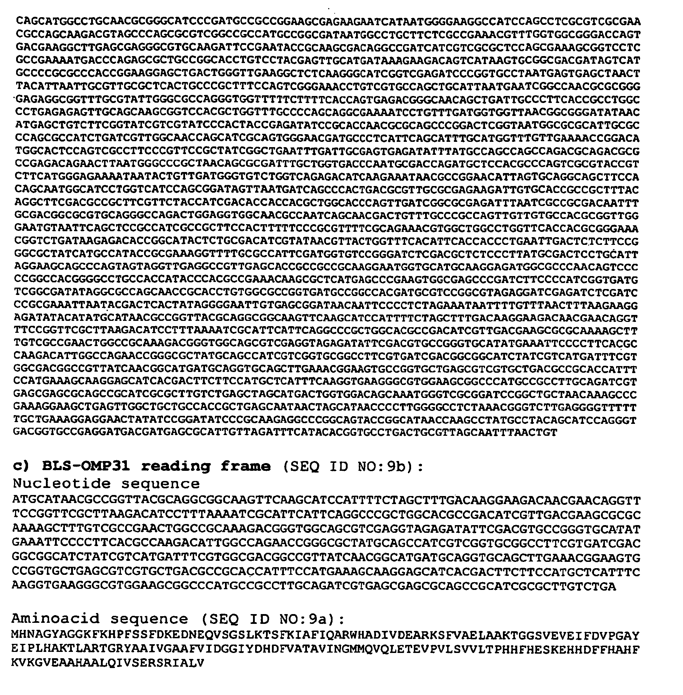

Figure 3 shows: a) the pET11a plasmid scheme including the BLS-OMP31 chimera sequence, showing the restriction sites used when cloning, b) the complete nucleotide sequence of the pET11a plasmid including the BLS-OMP31 chimera and c) the open framework of the BLS-OMP31 chimera.

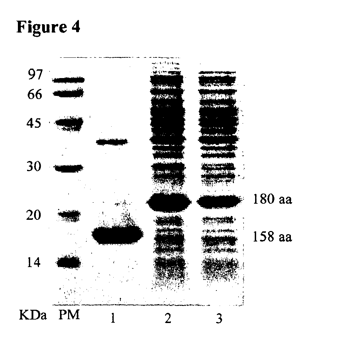

Figure 4 describes the expression of BLS-OMP31 analyzed by SDS-PAGE in a 17% acrylamide. MW: molecular weight markers in kilodaltons, 1: purified BLS, 2 and 3: aliquots of expression cultures of two BLS-OMP31 clones. The protein migrates like a 20-KDa monomer because the gel is denaturalizing.

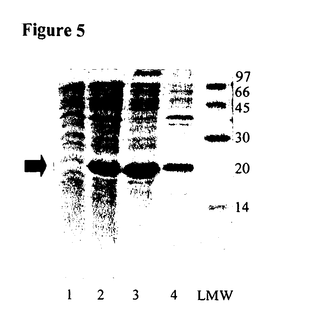

Figure 5 describes the expression of BLS-OMP31 analyzed by SDS-PAGE in a 17% acrylamide. 1: culture without induction, 2: culture induced with 1mM IPTG, 3: cytoplasmatic fraction, 4: fraction of inclusion bodies resuspended in 8M urea. The arrows show the bands corresponding to a BLS-OMP31. (LMWM = low molecular weight markers, weight indicated in kilodaltons).

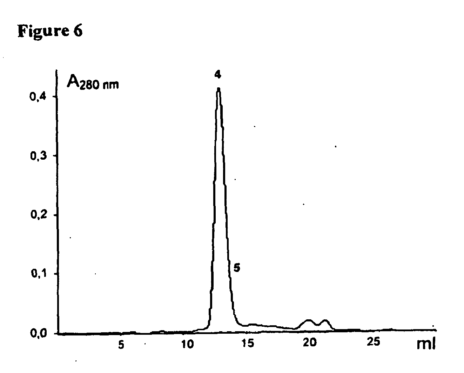

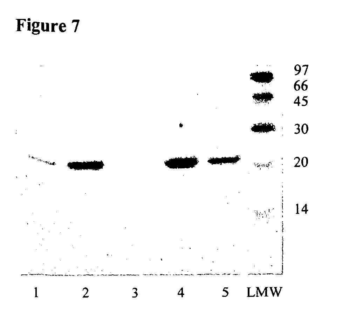

Figure 6 shows the chromatogram corresponding to the elution of the BLS-OMP31 in the Superdex 200 (Pharmacia, USA) column. The peaks were analyzed afterwards by gel and are numbered. *See* Fig. 7.
Figure 7 shows the purification data of BLS-OMP31 analyzed by SDS-PAGE in 17% acrylamide. 1 and 2: peaks obtained by a gradient of a BLS-OPM31 elution through a Q-Sepharose (Pharmacia, USA) column, 4 and 5: the numbering of the peaks correlates to the peaks in Figure 6. (LMWM = low molecular weight markers, weight indicated in kilodaltons).

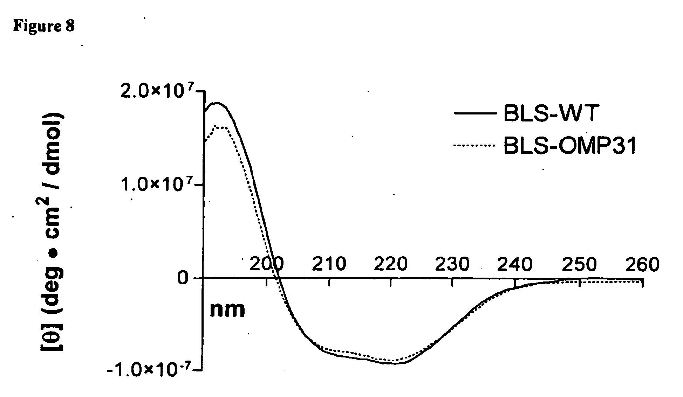

Figure 8 shows the circular dichroism spectra of BLS and the BLS-OMP31 chimera. The molar elipticity of a 1.0-µM solution of protein between 190 and 260 nm was monitored in a spectropolarimeter (Jasco, UK).

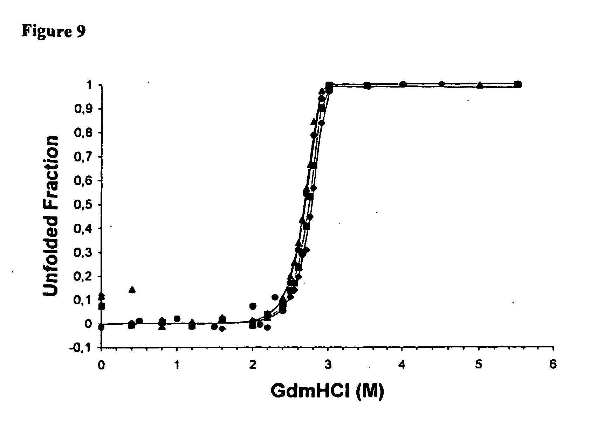

Figure 9 describes a comparative analysis of the stability of the BLS-OMP31 chimera in relation to the native protein. The curves show the sensitivity to unfolding in the presence of growing concentrations of the denaturalizing guanidine hydrochloride chemical agent, evaluated by the elipticity developed by the protein in a spectropolarimeter at 222 nm

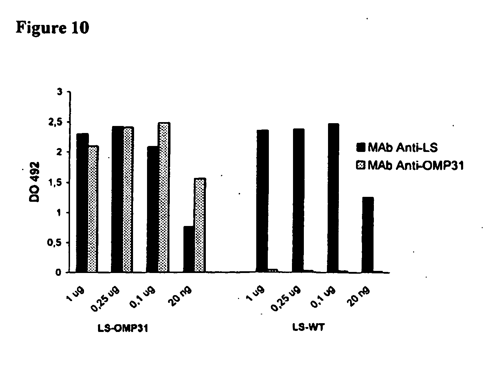

Figure 10 shows the antigenicity of BLS-OMP32 by ELISA. 1 µg, 0.25 µg, 0.1 µg and 20 ng of BLS-OMP31 and BLS were used as antigens. Anti-BLS Mab (1/1000) and Anti-OMP31 Mab (1/1000) were used as antibodies.

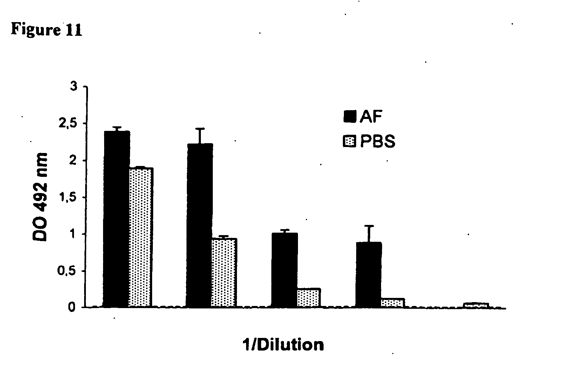

Figure 11 shows the immunogenicity of BLS-OMP31 analyzed by ELISA. The reactivity of 1/100 dilutions of sera from mice immunized with BLS-OMP31 with adjuvant ("AF") or without adjuvant ("PBS") against OMP31 are shown. Sera of each group were ordered per decreasing reactivity. The reactivity of a pre-immune serum (negative control) is illustrated by a dot line. A mouse from the AF group died before the extraction.

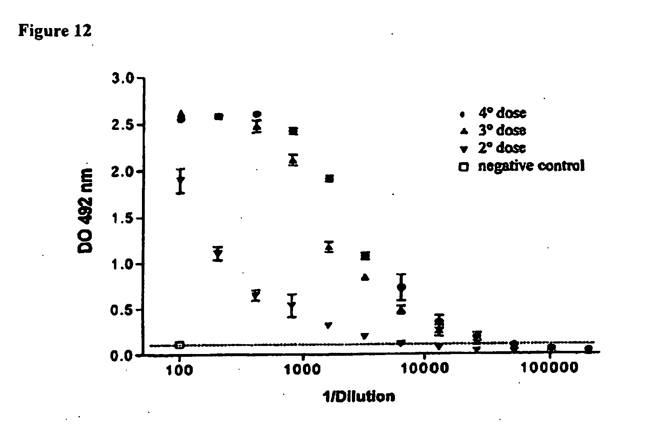

Figure 12 shows the titration results of an ELISA test of sera from anti BLS-OMP31 rabbit. The reactivity of the three sera against OMP31 was assayed. A 1/100 dilution of a rabbit anti native BLS serum was used as a negative serum. The reactivity of this serum is illustrated by a dot line.

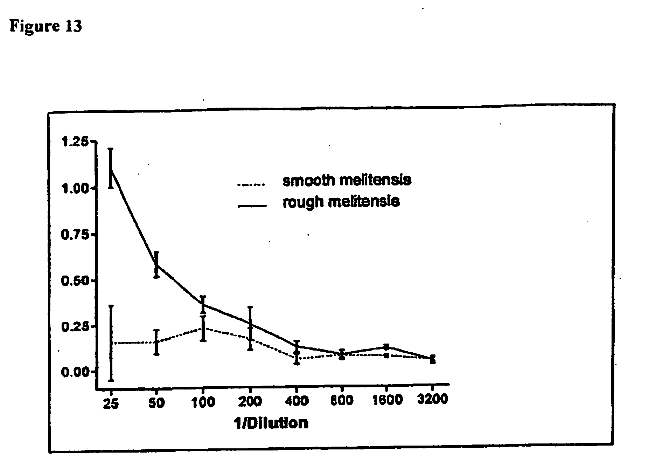

Figure 13 describes the reactivity of an anti BLS-OMP31 rabbit serum (4° dose) against smooth or rough *B. melitensis H38* whole bacteria. The reactivity corresponding to a rabbit anti native BLS serum, tittered simultaneously against both antigens, was substracted from the values shown. The errors correspond to the sum of mean standard errors.

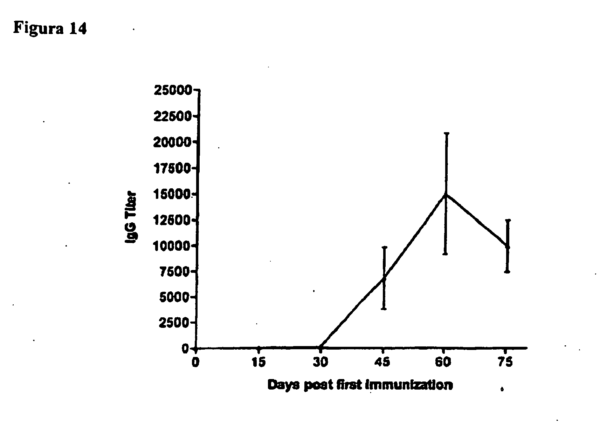

Figure 14 shows the kinetics of total anti-OMP31 IgG antibodies in mice batches BALB/c (8 per group) immunized in 4 occasions by intramuscular and intradermal route with 100 µg ofpCI-BLS-OMP31. Antibodies levels were analyzed by ELISA every 15 days. The values represent the mean value of the 8 animals ± S.D. The arrows show the time of the immunization. Sera of pCI mice (control) showed n DO values similar to or lower than the "cut-off" criteria.

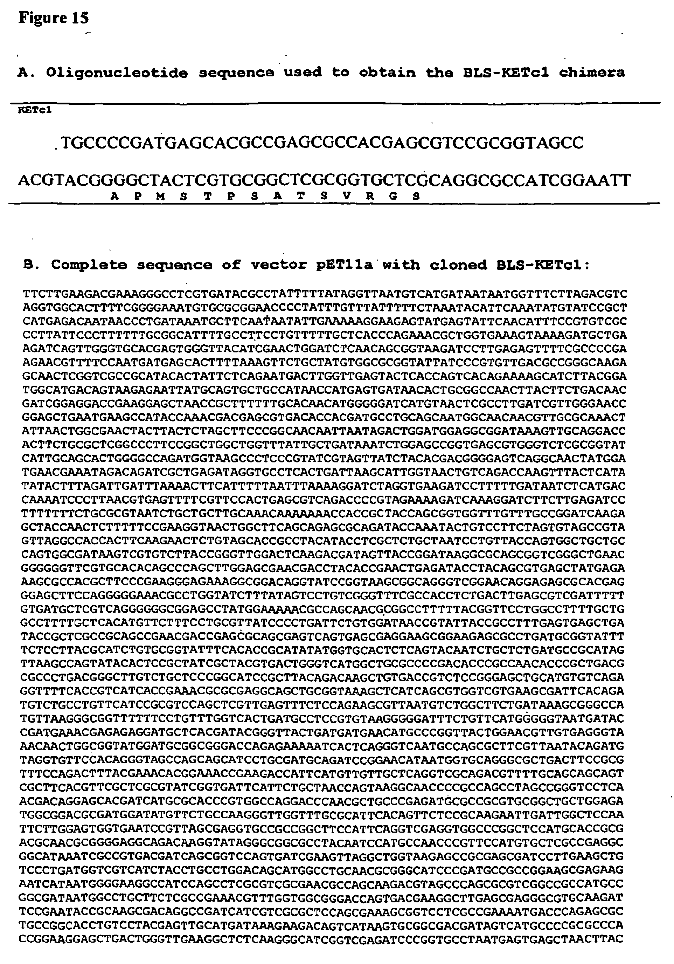

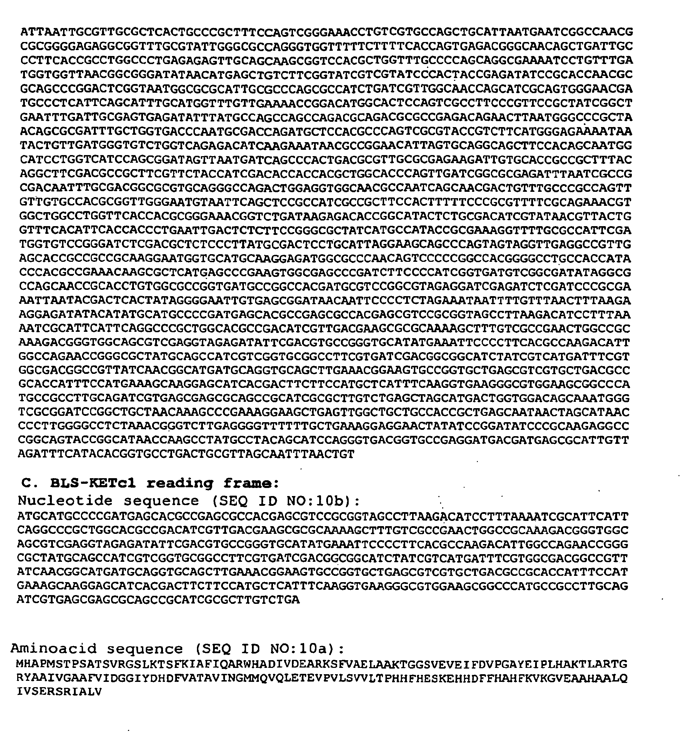

Figure 15 describes: a) the oligonucleotide sequence used to obtain the BLS-KETc1 chimera, b) the complete nucleotide sequences of the pET11a plasmid including the BLS-KETc1 chimera and c) the open framework of the BLS-KETc1 chimera and its amino acid sequence.

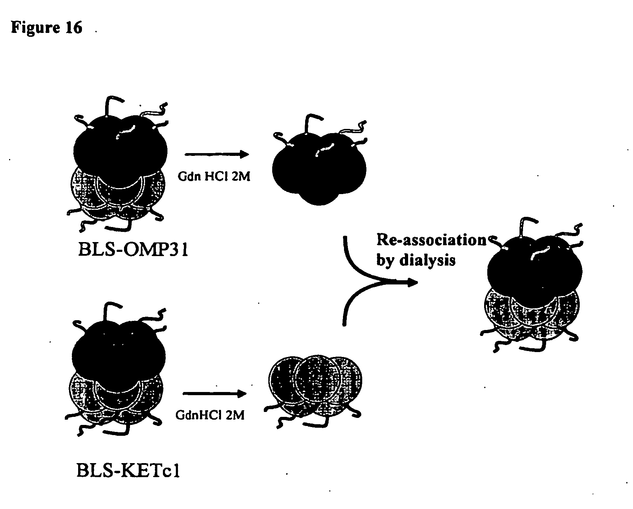

Figure 16 shows the general strategic scheme to produce mixed chimeras.

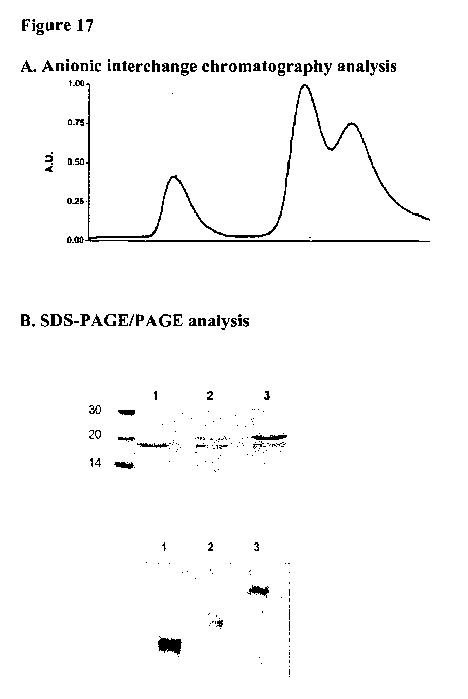

Figure 17 shows the purification results and analysis of mixed chimeras created between the BLS-OMP31 and BLS-KETc1 chimeras. A: analysis by anionic interchange chromatography in MonoQ column of BLS-OMP31 (peak 3) and BLS-KETc1 (peak 1) refolded chimeras and the stoichiometric mixture of both (peak 2), B1: analysis through SDS-PAGE of peaks 1, 2 and 3 obtained in the anionic interchange chromatography, B2: Analysis through native PAGE of pure BLS-OMP31 and BLS-KETc1 chimeras and of peak 2 corresponding to the mixed chimeras.

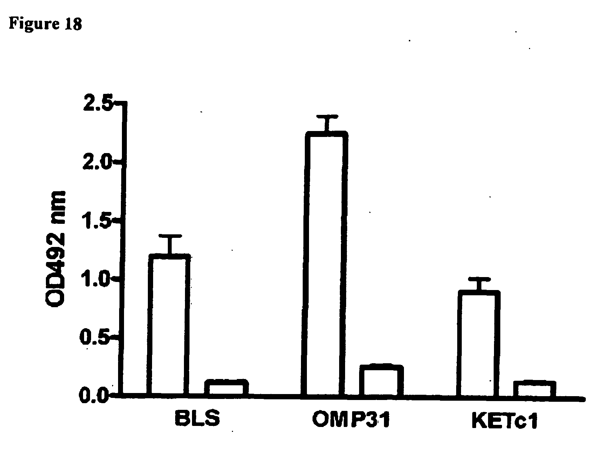

Figure 18 shows the immunogenicity of the BLS-OMP31-KETc1 mixed chimera analyzed by ELISA. Reactivity of 1/100 dilutions of sera from mice immunized with BLS-OMP31-KETc1 (empty bars) against BLS and against OMP31 and KETc1 synthetic peptides. The reactivity of a pre-immune serum (negative control) against the same antigens is indicated in gray bars.

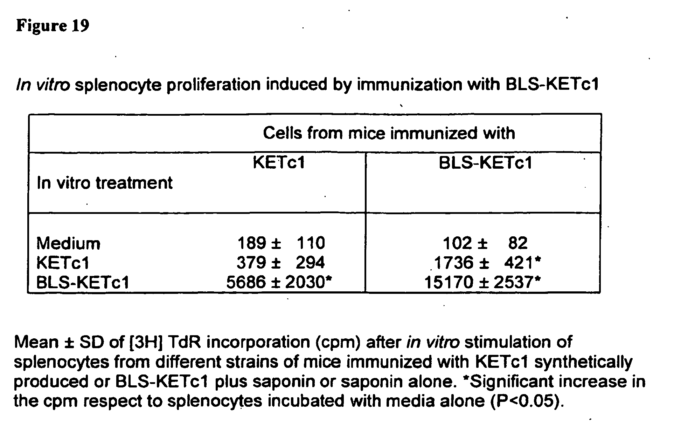

Figure 19 shows the *in vitro* proliferation of splenocytes induced by the immunization with BLS-KETc1. It indicates the average plus 1 standard deviation of the titrated (in cpm) timidine incorporation after the *in vitro* stimulations of splenocytes from mice previously immunized with KETc1 peptide or BLS-KETc1 emulsified in saponin. *Significant increase in cpm in respect of splenocytes incubated with a culture medium (P<0.05).

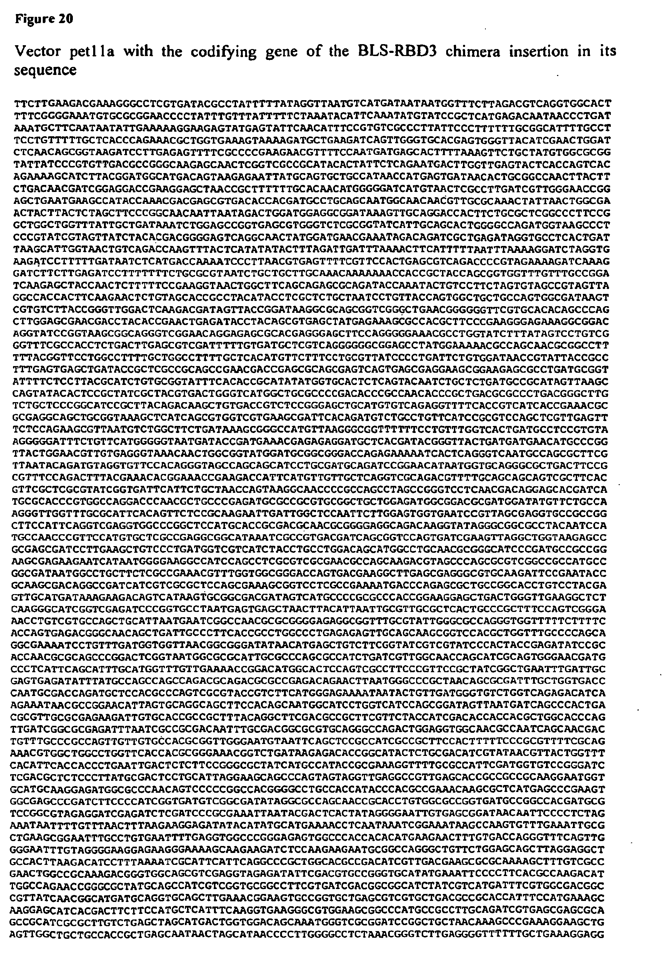

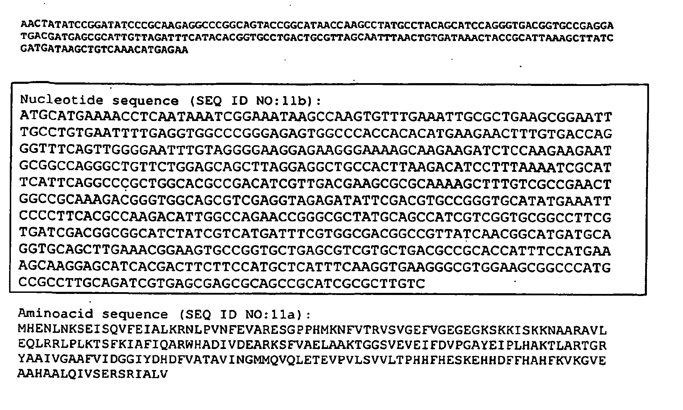

Figure 20 describes the nucleotide and amino acid sequence of the BLS-RBD3 chimera. The codifying sequence of domain RBD3 of the murine protein Staufen-1 is shown in red case. The codifying sequence of the truncated BLS in the first 8 residues of its amino termini is shown in black case.

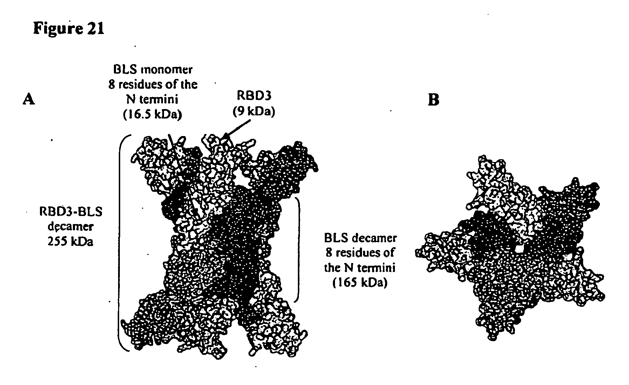

Figure 21 shows the structure of the BLS-RBD3 chimera (panel A: side view, panel B: upper view). The structure was designed with the MacroModel program merging the C-terminal end with the theoretical structure of the domain RBD3 of the murine protein Staufen-1 (shown in a blue range of colors) with the N-terminal end of the BLS crystallographic structure (Protein DataBank file pdb: IDIO) (shown in a red range of colors). The theoretical structure of domain RBD3 of the murine protein Staufen-1 was built by means of homology modeling with the structure of the domain RBD3 of the protein Staufen of *Drosophila melaganoster* (pdb: 1EKZ). The figure was built with the CHIMERA program.

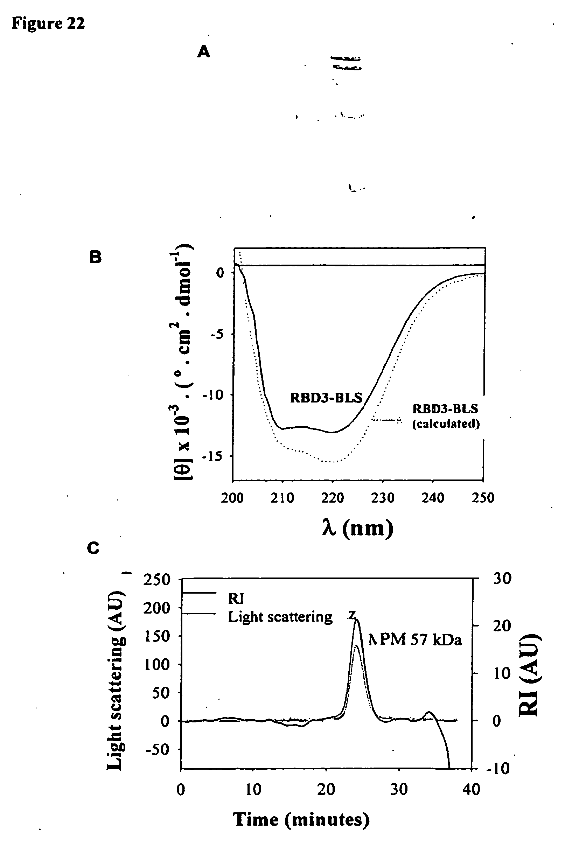

Figure 22 shows: A: the separation of the BLS-RBD3 chimera by SDS-PAGE in a 15% polyacrylamide gel (^{p}/ᵥ), various molecular weight markers were run in the right pathway. B: the circular dichroism spectrum in the UV far from the BLS-RBD3 chimera. The figure comparatively shows the chimera spectrum measured experimentally (in a continuous line) and its theoretical spectrum (in a dot line), calculated from the combination of the spectra corresponding to BLS protein and RBD3 domain of the murine protein Staufen-1 in isolation. The measurement was performed in a 50 mM Tris/HCl, 1.2 M NaCl, pH 8 buffer, at 4 °C. C: the assessment of molecular weight of the BLS-RBD3 chimera by using a light scattering detector connected in series to the outlet of a molecular filtering column and an refraction index (RI) detector. BLS-RBD3 was run in a Superdex-200 column and eluted with a 50 mM Tris/HCl, 3 M urea, 1 M NaCl, pH 8 buffer, at a flow of 0.5 ml/min. The elution was monitored by measuring its light scattering signal at 90 degrees and light refraction (LR). The sample molecular weight was calculated by comparing the relationship of its light scattering and refraction signals with those of a bovine seroalbumin sample used as a reference pattern (BSA molecular weight: 66.5 kDa).

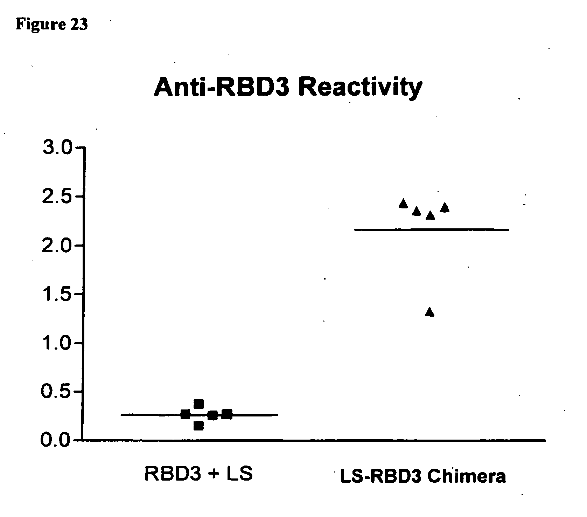

Figure 23 shows the optical density obtained at 492nm of a 1/100 dilution of sera analyzed for each mice of each investigational group. The result at 30-day interval after the second immunization is shown as a representative example. The horizontal line represents the mean value for each group.

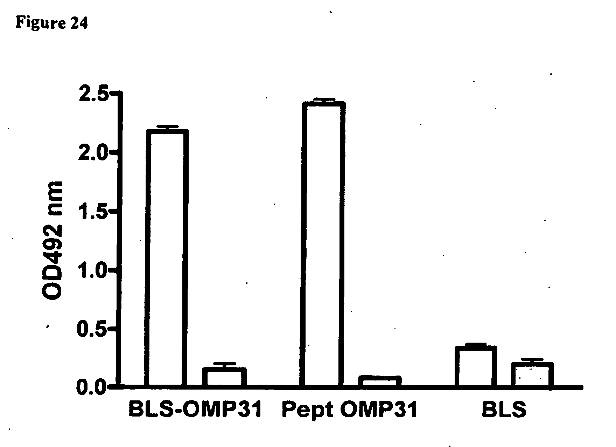

Figure 24 shows the results of an anti-OMP31 Mab ELISA test.

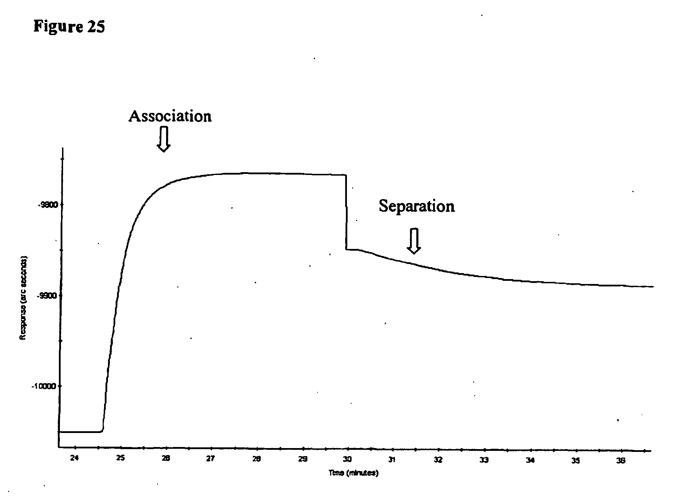

Figure 25 shows the detection of anti-OMP31, AcMo37F7 monoclonal antibody, using a biosensor formed by derivatization with the BLS-OMP31 chimeric protein.

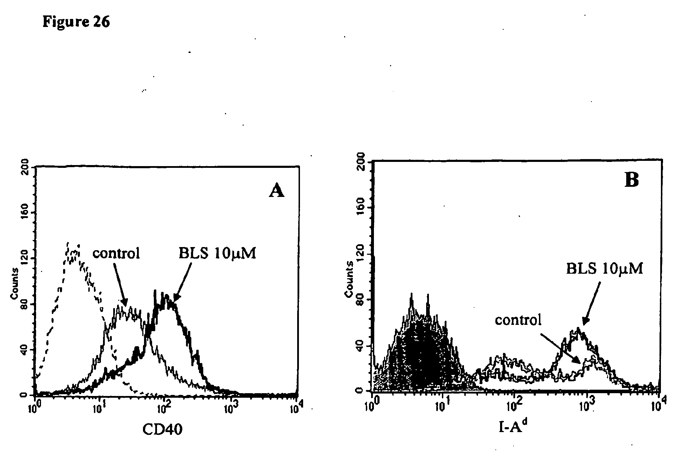

Figure 26 shows the expression of costimulating molecules in dendritic cells stimulated with BLS. Dendritic cells, derived from the bone marrow, were incubated during 18 hours with BSL (10µM BLS) or with medium only (control). The expression of CD40 in CD11c⁺ cells (A) and of I-A^{d} in CD11c⁺ (B) is shown. The isotype controls are shown to the left of each graph.

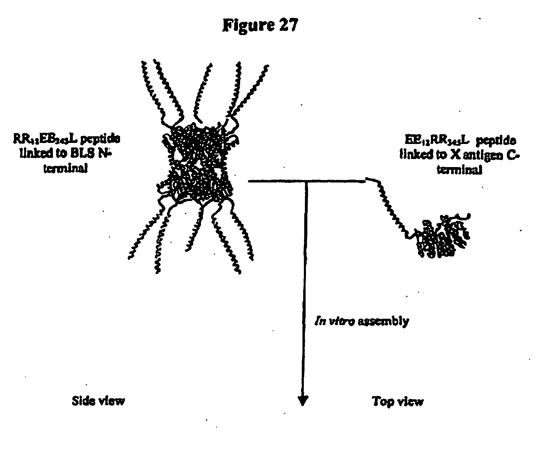

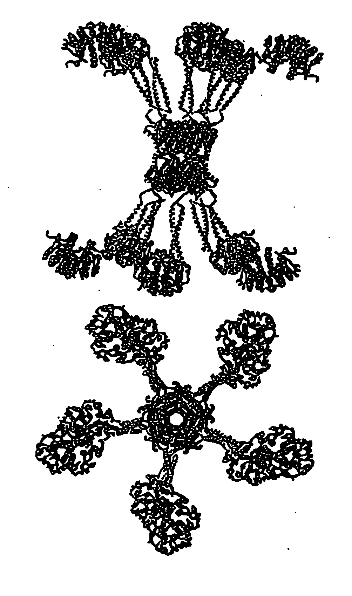

Figure 27 shows the strategy followed to form molecular assemblies with complementary heterodimeric peptides incorporated into the modified BLS protein and a theoretical X antigen target. *See* Braden, et al., *supra.* A Swisspdbviewer modeling software was used.

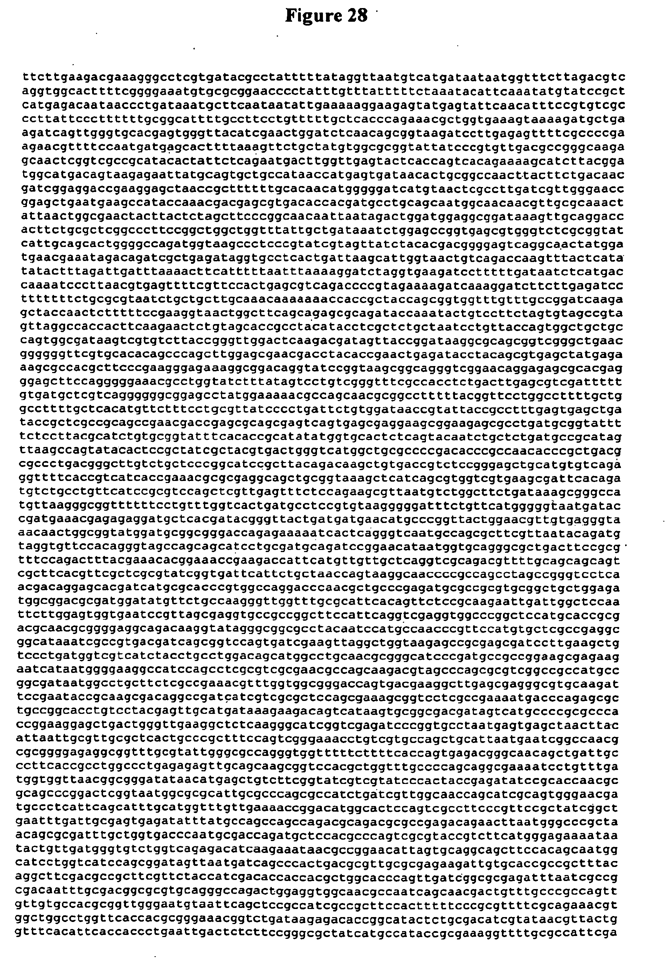

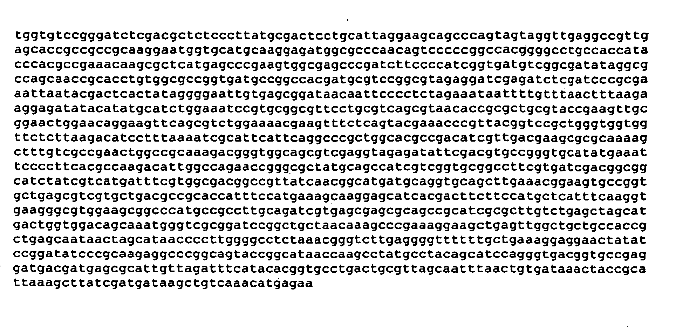

Figure 28. shows the nucleotide sequence of the BLS-RR₁₂EE₃₄₅L insert gene cloned in the expression vector pET11a (Novagen, USA). The section in red case shows the codifying region of the BLS-RR₁₂EE₃₄₅L insert peptide. The section in black case shows the codifying region for the modified BLS protein.

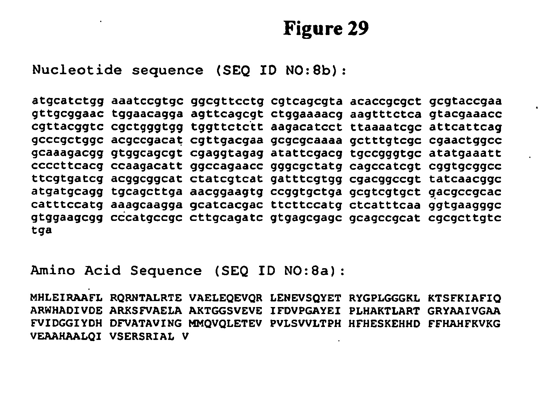

Figure 29 describes the nucleotide and amino acid sequences of the BLS-RR₁₂EE₃₄₅L fusion protein. The section in red case shows the codifying region of the RR₁₂EE₃₄₅L peptide. The section in black case shows the codifying region for the modified BLS protein. The section in green case shows the position of K₄₉ residue substituted with serine.

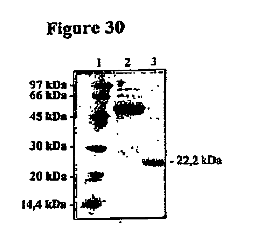

Figure 30 shows the separation of the BLS-RR₁₂EE₃₄₅L fusion protein by SDS-PAGE in a 17% polyacrylamide gel. 1: molecular weight markers, 2: irrelevant sample, 3: fusion protein BLS-RR₁₂EE₃₄₅L.

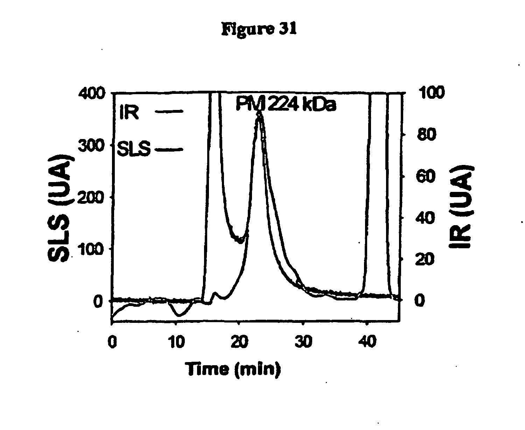

Figure 31 shows the assessment of molecular weight of the BLS-RR₁₂EE₃₄₅L chimera by using a light scattering detector serially connected to the outlet of a molecular filtering column and a refraction index (RI) detector. BLS-RR₁₂EE₃₄₅L was run in a Superdex-200 column and eluted with a 50 mM Tris/HCl, 3 M urea, 0.1 M NaCl, pH 8 buffer, at a flow of 0.5ml/min. The elution was monitored by measuring its light scattering signal at 90 degrees and light refraction (LR). The sample molecular weight was calculated by comparing the relationship of its light scattering and refraction signals with those of a bovine seroalbumin sample used as a reference pattern (BSA molecular weight: 66.5 kDa).

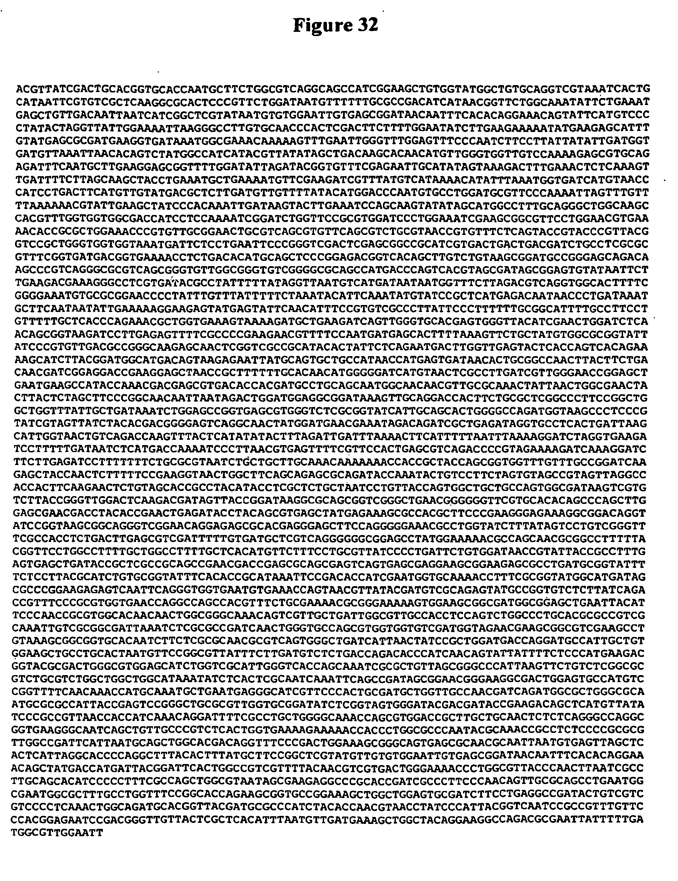

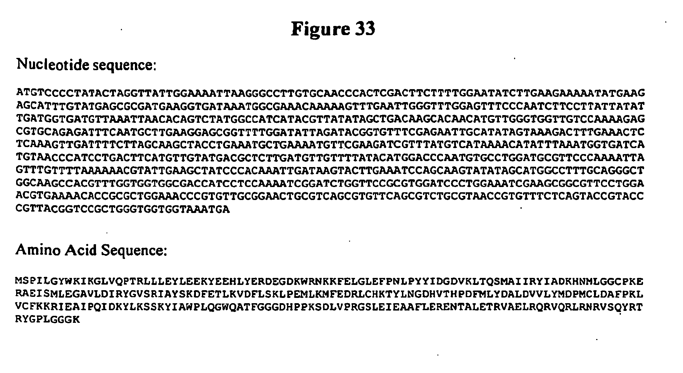

Figure 32 shows the nucleotide sequence of the peptide EE₁₂RR₃₄₅L insert gene cloned in the expression vector pGEX-4T1 (Novagen, USA). The sequence is inserted between the BainH1 y EcoRl restriction sites of the vector. The section in black case shows the codifying region for GST protein. The section in red case shows the codifying region for the peptide EE₁₂RR₃₄₅L.

### Description of the Invention

The present invention describes chimeric proteins useful, in general, for displaying peptides, polypeptides and proteins. The peptides, polypeptides and proteins shown herein may induce an immune response or be suitable for other useful purposes.

Object of the present invention are the isolated chimeric proteins according to claim 1.

The present invention also describes pharmaceutical compositions and vaccines of high immunogenic value and efficacy. These compositions and vaccines include the chimeric proteins according to claim 1.

The chimeric proteins described in this application can display peptides, polypeptides, proteins or molecules of other distinctive pathogens and non-pathogens types.

More specifically, the chimeric proteins described in this application can be useful for the treatment and prevention of human diseases. These entities can be used for the inoculation of antigens, toxins and protein domains with low or without immunogenic activity. Examples of these indications would be the vaccination against common measles, German measles (rubella), hepatitis, tetanus, pertussis, poliomyelitis, diphtheria, mumps, meningitis and rabies in infants. Other examples of these indications are the vaccination against hepatitis A, B and C, influenza, encephalitis, rabies, typhoid fever, yellow fever, herpes simplex, varicella zoster, dengue, human papillomavirus, cholera, malaria, tuberculosis and mumps in adults. Other examples of these indications are vaccines useful to counteract bioterrorism, for the following pathogens: *Botulinum toxin, Bacillus anthracis, Clostridium perfringens, Bacillus subtilus, Bacillus thuringiensis,* hemorrhagic conjunctivitis virus (Enterovirus 70) and rotavirus.

These entities could also be used for the treatment of chronic conditions, such as Alzheimer's disease, Parkinson's disease and rheumatoid arthritis or other conditions, such as allergies and tumors. An example of the latter could consist of a chimeric protein simultaneously including antibodies or fragment of antibodies against tumoral markers or radioactive elements for radiotherapy. The chimeric proteins developed according to the present invention, and the antibodies derived from them, could also be used for the diagnosis of fertility and pregnancy, or of diseases such as colon, lung, breast and prostate cancer. These entities could also be used to monitor and control drug abuse or the progress of therapeutic treatments.

The chimeric proteins described in the present invention also have multiple uses for the breeding of domestic animals, farm animals and fish. These entities may be used to prepare vaccines against *Brucella spp.,* a bacterial agent that causes many problems in cattle, or *against Piscirickettsia salmonis,* which affects mainly the commercial breeding of salmon. Moreover, these entities can be useful to administer antibacterial agents, such as penicillin, amoxicillin or other penicillin subproducts, alone or in combination with specific antibodies to domesticated animals, like cats and dogs, or farming animals, like cattle.

Other possible uses of the chimeric proteins described in this application are the preparation of vaccines against *Mycoplasma hyopneumoniae,* a pneumonic agent that produces great losses in pigs, and the administration of parasiticides, like albendazole, fenbendazole and ivermectin against *Ostertagia ostertagi,* to calves and cattle in general. It would also be possible to use these new entities to administer parasiticides, like abamectine and praziquantel, to horses, and to vaccinate with epitopes of antigens or viral protein domains, like poultry influenza, to birds. In all cases, the chimeric protein can simultaneously contain the parasiticide agent and the specific antibodies against the involved parasite. An additional therapeutical use of the new entities would be the administration of antinflammatory agents, like carprofen to domesticated animals, like dogs and cats.

The chimeric proteins according to the present invention could also have different indications for the control of pathogens. These entities could be used to modify the expression of certain hormones to accelerate the growth rate and increase the production of milk, in farming animals, or make their meat leaner. An example of these non-conventional indications would be the use of these entities for immunocastration of farming animals, like pigs.

The chimeric proteins described in this application can also be used for the large-scale production of vaccines and antibodies in molecular farming. Under this approach, the vaccine or antibody will be expressed firstly in a suitable plant. The vaccine or antibody will then be extracted from the plant and purified to prepare a pharmaceutical formulation. Another possibility would be to feed animals with plants transformed with the entities described herein in order to immunize via their food intake (*edible vaccines*)*.*

A particular advantage of the present invention is that the vehicles described herein are able to carry peptides larger than other vehicles known in the art, such as the "*virus like particles*". This advantage is due to their small size and higher stability. This antigenic display system of the vehicles described herein has the additional advantage of displaying peptides, polypeptides and proteins inserted repeatedly and ordered spatially, increasing their stability and half-life. The carrier protein (BLS) also has a considerable adjuvant effect on peptides, polypeptides and proteins inserted thus enhancing the immune response effectiveness.

Another advantage of the present invention is that the carrier protein BLS induces an immune response by itself without the presence of additional adjuvants. This characteristic facilitates the administration of vaccines prepared according to the present invention by various routes of administration (e.g. intravenous, nasal, oral, needle- free) with or without adjuvants.

The pharmaceutical compounds and vaccines described in this application are characterized by their high stability at room temperature. This characteristic would likely preserve the compound or vaccine without keeping a strict cold chain of storage.

The creation of mixed chimeras with multiple peptides inserted in the different ends of the carrier protein BLS is also useful to design polyvalent vaccines or vaccines aimed at different routes of immune response. The prevailing opinion in the art at present is that a vaccine should not contain more than 10 immune response-inducing agents. It is also the prevailing opinion in the art that the vaccine should contain 5 or less inducing agents to achieve optimal results.

The chimeric proteins described in this application are useful for the production of antibodies. These antibodies and their associated entities could be used in diagnosis. It is also possible to develop kits for the diagnosis of diseases using the above-mentioned antibodies and associated entities.

Several of these entities developed according to the present invention could be used also to display peptides and build protein libraries and their associated applications (e.g., combinational biology applied to the identification of molecules for the development of drugs or the identification of polypeptide sequences binding preferably inorganic compounds, for nanobiological applications). Some of these new entities could also be used for the production and assembly of nanotechnologic devices or for the conduction of nanotechnologic processes.

The chimeric proteins according to the present invention could be used in the construction and development of biosensors applied to the analytical detection of several substances and molecules (e.g., detection ofcontaminants or toxins in water, soil or air, detection of residues of drugs, herbicides and pesticides in food).

Another advantage of the entities described in this present application is its easy and low-cost production. The chimeric proteins described in this application are obtained in high amounts from a model of transformed strains from *E*. *coli,* a microorganism of well-known handling and culture. The proteins of interest obtained according to this method are easily purified from the culture medium.

The isolated chimeric proteins claimed herein are formed by a peptide, polypeptide or protein linked to a protein mutated from the lumazine synthase of *Brucella spp.* whose codifying nucleotide sequence has been modified in its first 8 residues at its N-termini to allow its cleavage by restriction enzymes that do not cleave naturally the codifying nucleotide sequence of the native lumazine synthase protein of *Brucella spp.* The codifying nucleotide sequence of the mutated lumazine synthase of *Brucella spp.* protein has been modified in its first 8 residues at its N-termini in order to allow its cleavage with the Nsi I and Afl II restriction enzymes. Preferably the mutated lumazine synthase proteins used according to the present invention have the amino acid sequence SEQ ID NO:7a. The linked peptide, polypeptide or protein can be homologous or heterologous. In a version of the present invention, the peptide linked to the mutated protein is a heterodimerization domain. Preferably, this heterodimerization domain is a "leucine zipper" domain. The chimeric proteins thus obtained include, but are not limited to, the amino acid sequence SEQ ID NO:8a and are used preferably for the coupling of protein domains, complete proteins or other non-protein entities. In another embodiment of this invention, the combinations of isolated chimeric proteins described in this present application are also claimed. The uses of these isolated chimeric proteins and of their combinations are also claimed.

In another embodiment of the present invention, the isolated codifying nucleotide sequences for the chimeric proteins described in this application are claimed. These sequences can be of RNA, genomic DNA or copy DNA. In another embodiment of the present invention, the codifying sequence for the linked peptide, polypeptide or protein is located in the 5' region of the codifying nucleotide sequence for the mutated lumazine synthase protein of *Brucella spp.* In another embodiment of the present invention, the codifying sequence for the linked peptide, polypeptide or protein is operatively linked to the 5' region of the codifying nucleotide sequence for the mutated lumazine synthase protein of *Brucella spp.* Preferably the following DNA sequences utilized are: SEQ ID NO:1b, SEQ ID NO:2b, SEQ ID NO:3b, SEQ ID NO:4b, SEQ ID NO:5b, SEQ ID NO:6b, SEQ ID NO:7b. Specific instances of the mutated protein sequences used in the present invention include, but are not limited to, the framework nucleotide sequences: a) BLS-OMP31 b) BLS-KETc1 and c) BLS-RBD3, used in Examples 1, 5 and 8, respectively. In another embodiment of this invention, the combinations of isolated nucleotide sequences described in this present application are claimed. The uses of these isolated nucleotide sequences and their combinations are also claimed.

In another embodiment of the present invention, the combinations of said isolated chimeric proteins and the isolated nucleotide sequences described in the present invention are claimed.

In another embodiment of the present invention, the vectors including the codifying sequences for the chimeric proteins described herein are claimed. These vectors can be bacterial, viral or other origin, and are able to express, or facilitate the expression of the chimeric proteins described in the present application. Specific instances of these vectors are pBLS-OMP31, pBLS- KETc1 and pBLS-RBD3 plasmids, used in Examples 1, 5 and 8, respectively. Preferably, the pBLS-OMP31, DSM 15546 plasmid is used as a vector and as a precursor for the generation of plasmids expressing the chimeric proteins described in the present application. The uses of these vectors are also claimed.

In another embodiment of the present invention, the cells or microorganisms transformed with the vectors described in this application are claimed. These microorganisms could be of prokaryotic, eukaryotic or other origin. Specific instances of the cells that can be transformed with the vectors described in the present invention include, but are not limited to, insect cells, bacteria such as *Escherichia coli,* and mammal cells such as CHO, COS, BHK, Namalwa and HeLa More preferably, competent strains of *Escherichia coli* are used for such transformations. The uses of these cells are also claimed.

In a version of this present, the isolated chimeric proteins described herein are able to induce an immune response in a eukaryotic organism These chimeric proteins can induce cellular or humoral responses in the treated organism In these cases, the response could be against the same antigen, toxin, protein domain or inducing agent or against different antigens, toxins, protein domains or inducing agents. The antigens, toxins, protein domains or agents used according to the present invention could be of bacterial, parasitic, viral or other origin, and be able to induce an immune response. Specific instances of these inducing agents include, but are not limited to the: a) the 27 amino acid sequence of the OMP31 protein of *Brucella mellitus*, b) the 14 amino acid sequence of the KETc1 protein of *Tenia solium* and c) the 75 amino acid sequence of the RBD3 domain of murine protein Staufen, used in Examples 1, 5 and 8, respectively. In general, the treated eukaryotic organisms are birds, fish or mammals. Preferably, these organisms are from a murine, rabbit or human origin. More preferably, the organism is of human origin. The uses of these chimeric proteins able to induce an immune response are also claimed.

In a version of this present, the codifying nucleotide sequences for the isolated chimeric proteins described herein are able to induce an immune response in a eukaryotic organism These nucleotide sequences can induce cellular or humoral responses in the treated organism. In these cases, the response can be against the same antigen, toxin, protein domain or inducing agent or against different antigens, toxins, protein domains or inducing agents. The antigens, toxins, protein domains or inducing agents used according to the present invention can be of bacterial, parasitic, viral or other origin, and are able to induce an immune response. Specific instances of these inducing agents include, but are not limited to, the codifying nucleotide sequences for: a) the 27 amino acids of the OMP31 protein of *Brucella melitensis,* b) the 14 amino acids of the KETc1 protein of *Tenia solium* and c) the 75 amino acids of the RBD3 domain of murine protein Staufen, used in Examples 1, 5 and 8, respectively. In general, the treated eukaryotic organisms are birds, fish or mammals. Preferably, these organisms are from a murine, rabbit or human origin. More preferably, the organism is of human origin. The uses of these nucleotide sequences able to induce an immune response are also claimed.

In a version of the present invention, pharmaceutical compositions or vaccines including the following are claimed: 1) at least one type of the chimeric proteins described in this application, 2) at least one type of the codifying nucleotide sequences for the chimeric proteins described in this application or 3) a combination of 1 and 2).

The pharmaceutical formulations or vaccines claimed in the present application can be in a liquid state or in any other pharmaceutical form known in the art, such as injectable emulsions. The pharmaceutical compositions or vaccines described in the present invention can also be in tablets, liquid solutions, suspensions or elixirs for oral administration or in sterile liquids such as solutions or suspensions. Preferably an inert medium is used, such as saline media, phosphate-saline buffers and any other medium where the chimeric proteins, nucleotide sequences or segments thereof have a proper solubility.

The active agents of the pharmaceutical compositions or vaccines claimed in this invention are present in effective physiological doses. These active agents can be administered alone o in combination with acceptable pharmaceutical excipients, such as adjuvants, in order to increase the production of antibodies.

The pharmaceutical compositions or vaccines used according to the present invention include, but are not limited to, several oily formulations such the Freund adjuvant, tyrosin stearate, MDP dipeptide, saponin, aluminum hydroxide (alum), lymph cytokines, the native protein of the lumazine synthase of *Brucella spp.* and proteins mutated from the lumazine synthase of *Brucella spp.* described herein. *See* US 4,258,029 (Moloney, et al.); US 5,057,540 (Kensil, et al.). Preferably tyrosin stearate, aluminum hydroxide, the native protein of the lumazine synthase of *Brucella spp.* and the mutated proteins described herein are used in the case of human beings. The Freund adjuvant, though powerful, is not used usually in human beings. The pharmaceutical composition or vaccine can also be administered using slow releasing mechanisms, such as liposomes. *See* US 4,235,877 (Fullerton, W.). The preparation of vaccines and pharmaceutical compositions for their use in higher organisms is known in the art. *See* Remington, et al., Pharmaceutical Sciences, Mack Publishing Co., Easton, Pennsylvania, 1980; Voller, et al., Eds., New Trends and Developments in Vaccines, University Park Press, Baltimore, Maryland, 1978.

The present invention allows to induce an immune response in a eukaryotic organism. The relevant method encompasses the administration to such organism of an effective amount of a pharmaceutical compound or vaccines including: 1) at least one type of the chimeric proteins described in this application, 2) at least one type of the codifying nucleotide sequences for the chimeric proteins described in this application or 3) a combination of 1 and 2). In a version, a humoral response is induced. In another version, a cellular response is induced. In another version, humoral or cellular responses are induced simultaneously or sequentially. In these cases, responses can be against the same antigen, toxin, protein domain or inducing agent or against different antigens, toxins, protein domains or inducing agents. The antigens, toxins, protein domains or agents used according to the present invention can be of bacterial, parasitic, viral or other origin, and are able to induce an immune response. Specific instances of these inducing agents include, but are not limited to, the nucleotide sequences codifying for or to the amino acid sequences of: a) the 27 amino acids of the OMP31 protein of *Brucella melitensis,* b) the 14 amino acids of the KETc1 protein of *Tenia solium* and c) the 75 amino acids of the RBD3 domain of murine Staufen protein, used in Examples 1, 5 and 8, respectively. Examples of organisms that may be treated with the vaccines and pharmaceutical compounds described in this application are birds, fish or mammals. Preferably, these organisms are from a murine, rabbit or human origin. More preferably, the organism is of human origin. The vaccines or pharmaceutical compositions described in the present invention can be administered in one or several doses. Preferably, the administration is performed in only one dose. The administration can also be performed by subcutaneous, intravenous, intramuscular, oral, nasal or needle-free route. Preferably, it is performed by subcutaneous or intramuscular route.
The monoclonal and polyclonal antibodies produced in response to the immunization with said chimeric proteins and the nucleotide sequences are described herein. The hybridomas developed for expressing and producing the antibodies described in this application are also described. The uses of these antibodies for their preventive, therapeutic, diagnostic and other purposes are described. The pharmaceutical compounds including said antibodies and their uses are also described.

In another embodiment of the present invention, the protein conjugates formed between the chimeric proteins described herein and at least one ligand are claimed. In a version of this invention, the link between the chimeric protein and the ligand is covalent. In these cases, the link is preferably peptidic. In another embodiment of this invention, the link between the chimeric protein and the ligand is non-covalent.

In another embodiment of the present invention, the typical quaternary structure of the chimeric proteins described herein is claimed.

As used herein, the term "active agent" is defined as: 1) genomic DNA, copy DNA, messenger RNA or segments of thereof, codifying for the chimeric proteins, or segments thereof, described in the present application and 2) the chimeric proteins, or segments thereof described in the present application, 3) the combinations of 1) and 2) or 4) the protein conjugates formed between the chimeric proteins described herein and other entities, segments thereof.

As used herein, the term "effective amount" is defined as a quantity sufficient to produce one or more of the following results: 1) induction of a proper immune response, whether humoral or cellular, including the production of antibodies against the inducing agent; 2) the inhibition of the growth, development, size or motility of the cell or microorganism associated with the inducing agent. When the inducing agent is a tumor, the "effective amount" will be the quantity sufficient to reduce the size, to prevent growth, to inhibit the metastasis or tumor growth, or to relieve the discomfort caused by such tumor or to prolong the life of an individual suffering from such tumor.

As used herein, the term "mammal" is defined as a hot-blooded vertebrate animal whose descendants are fed with milk secreted by its mammal glands. The term "mammal" includes, but it is not limited to, rats, mice, rabbits, dogs, cats, goats, cows, sheeps, pigs, primates and human beings.
As used herein, the term "pharmaceutical composition or vaccine" is defined as a diluent, adjuvant or excipient with which the active agent is administered jointly. It includes each and every solvent, dispersion media, aqueous and gaseous solutions, coatings, antibacterial and antifungal agents, isotonic agents, retardants or catalysts of absorption or similar substances. The use of such media and agents in the administration of active pharmaceutical substances is known in the art. The use of such conventional media with the active agent is indicated unless the active agent is rendered ineffective by the media. Supplementary active principles can also be incorporated into the active agents described in the present invention. The term "pharmaceutical compositions or vaccines" include, but are not limited to, inert solvents or excipients, sterile aqueous solutions and several non-toxic organic solvents. The "pharmaceutical compositions or vaccines" should neither react with nor otherwise reduce the efficacy or stability of the active agent. The acceptable pharmaceutical vehicles include, but are not limited to, water, ethanol, polyethileneglycol, mineral oil, petrolatum, propyleneglycol, lanolin and similar agents. The "pharmaceutical compositions or vaccines" for injectable use include sterile aqueous solutions (when soluble in water) or sterile dispersions and powders for preparing sterile injectable dispersions or solutions. In every case, the formulation should be sterile and preferably fluid in order to enable its dispensing through a syringe. It should also be stable under manufacturing and storing conditions and should be protected from the contaminating effect of microorganisms such as bacteria, virus and fungi.

As used herein, the term "preventive use" is defined the capacity to induce and generate an immune response against one or more antigens, toxins, protein domains or other inducing agents, or segments thereof.

As used herein, the term "sequential administration" means that: 1) the same active agent is administered in more than one occasion at consecutive periods of time or 2) two or more active agents are administered alternatively in more than one occasion at consecutive periods of time. When the administration is "sequential", the time difference between the administrations of active agents may be minutes, hours, days, weeks or months depending on whether the use is preventive or therapeutical and on the nature of the treated organism.

As used herein, the term "single or simultaneous administration" means that one or more active agents are administered in the same occasion at once.

As used herein, the term "therapeutic use" is defined to refer to every process, application, therapy or similar action, in which a higher organism, including a human being, is subject to medical care with the aim of improving such organism condition or resistance to diseases, whether directly or indirectly.

### Example 1

### BLS-OMP31 Chimeric Gene Construction

This example describes the strategy used to insert the sequence corresponding to the 48 to 74 amino acids of the polypeptidic sequence of the OMP31 protein of *Brucella melitensis* into the 10 amino termini of the decamer forming the lumazine synthase *of Brucella spp.*

### 1. Mutation and Cloning

The pBLS-OMP31 plasmid, SEQ ID NO:9c, was constructed through the following protocol:
a) To clone the codifying gene for the lumazine synthase of *Brucella spp* (BLS), the BLS sequence was obtained by PCR amplification with specific primers from the genomic DNA of *B. abortus* and cloned in the pET11a vector (Novagen, USA). The pET11-BLS plasmid including the open reading frame of the lumazine synthase of *Brucella spp.* was digested further with the Bam HI and XbaI restriction enzymes. The insert obtained was subcloned in a pALter-Ex1 vector (Promega, USA).
b) A directed mutagenesis was performed over the sequence codifying for the open reading frame of the lumazine synthase of *Brucella spp* (BLS). This sequence was cloned in the pALter-Ex1 vector (Promega, USA) using an ALTERED sitesII kit (Promega, USA). In order to develop the cassette, two new restriction sites were inserted in the 5' region of the BLS gene: an Nsi site in the first two codons of the 5' end and one AFL II site in the two codons comprising the 8 and 9 residues of the native amino acids sequence of BLS. It was taken into consideration that these restriction sites do not occur either in the native BLS gene or in the pET11a vector. *See* Figure 1.
c) The mutation was checked afterwards by sequencing. The cassette including the mutated BLS sequence (SEQ ID NO:1b) was subcloned in the pET11a vector (Novagen, USA) to obtain the plasmid called hereinafter pBSLm
d) To insert the sequence corresponding to the OMP31 plasmid, two oligonucleotides were designed so that they would form a double-stranded DNA and include the codifying sequence for the 48-74 amino acids of the OMP31 protein of *Brucella melitensis* protected by the cohesive ends typical of the Nsi I and Afl II restriction enzymes when annealing. Figure 2 shows the oligonucleotides designed for constructing the pBLS-OMP31 and the synthetic insert formed by these.
e) The pBLSm plasmid was digested with the Nsi I and Afl II restriction enzymes. The codifying sequence corresponding to the first 8 residues of the BLS was removed. The original BLS sequence was changed for the nucleotide sequence of the inserted OMP31 peptide in this case.
f) The synthetic insert of step d) above was linked to the open pBLSm cassette obtained in step e) by incubating overnight with DNA T4 ligase enzyme at 16°C. The insertion was confirmed by sequencing. Thus, a gene with the SEQ ID NO:9b sequence was obtained. The sequencing analysis showed that the first 8 amino acids of the lumazine synthase of the *Brucella spp.* were replaced by the 27 amino acids from the 48-74 sequence of the OMP31 protein of *Brucella melitensis.* This open reading frame was called the BLS-OMP31 chimera, of SEQ ID NO:9b. Its corresponding plasmid was called pBLS-OW31, of SEQ ID-NO:9c. *See* Figures 3b and c.

This experience was repeated using the DNA sequences of the mutated BLS SEQ ID NO:2b, SEQ ID NO:3b, SEQ ID NO:4b, SEQ ID NO:5b, SEQ ID NO:6b and SEQ ID NO:7b. Similar results were obtained.

### 2. Transformation

A competent strain of *E*. *coli* BL21 (DE3) bacteria was transformed by thermal shock with the pBLS-OMP31 plasmid obtained according to the protocol above. Afterwards, bacteria were cultured in agar plates including LB-agar/ampicilin to choose those transformed with the plasmid. 2 ml of a LB/ampicilin medium was inoculated to a colony extracted from agar plates for small-scale expression tests. The colony was shaked and incubated at 37°C. The saturated culture was induced with 2 µl of 1M IPTG. Three hours later, 100 µl of culture was removed and centrifuged. The resulting pellet was resuspended in 25 µl of sample buffer IX for its analysis by SDS-PAGE 17%. *See* Figure 4.

### 3. Expression and Purification of the BLS-OMP31 Chimeric Protein

A 5-ml preculture of the transformed strains was cultured to saturation according to the step above with 500 ml of LB/ampicilin. The culture was incubated and shaked at 37 °C. It was induced with 0.5 ml of IPTG (1M) to reach an optical density of 0.6-0.8 at 600 nm The culture was removed three hours later and was centrifuged at 4,000 g for 20 minutes. The pellet was resuspended in 15 ml of suspension solution (50 mM Tris, 5 mM EDTA, 1% Triton X-100, pH 8.0).

The suspension was sonicated at 1 minute intervals every minute for 5 minutes and was centrifuged at 20,000 g for 30 minutes. The pellet was resuspended in 15 ml of suspension solution without Triton X-100 and the sonicate process was repeated. The procedure was repeated for a third time. The chimera expressed in the cytoplasm was contained in three sonicate supernatants while inclusion bodies were contained in the pellet. *See* Figure 5.

The inclusion bodies were resuspended in PBS buffer with 8 M urea and were left overnight at room temperature. The resuspension was dialyzed against PBS for two days with a buffer change. The sample was centrifuged and the supernatant was dialyzed against buffer A (50mM Tris/HCl, pH 8.5). The first purification step was performed by anionic interchange chromatography in a MonoQ or a Q-Sepharose (Pharmacia, USA) column in a FPLC equipment (Pharmacia, USA). The sample was injected in the balanced column with buffer A and was eluted by linear gradient up to 50% of buffer B (buffer A + 1 M NaCl).

The purification second step was performed by chromatography in a Superdex 200 (Amersham, UK) molecular exclusion column. *See* Figures 6 and 7. For this, the chimera peak was concentrated in a Centriprep (Millipore, USA) tube and injected in the column for elution with PBS. The presence of the chimeric protein in the peaks was evaluated by SDS-PAGE.

The construction of the BLS-OMP31 chimeric gene was performed by using molecular biology techniques known in the art. *See* Sambrook, et al., Molecular Cloning: A Laboratory Manual, Cold Spring Harbor Laboratory Press, New York, New York, 1989; Brown, Gene Cloning, Chapman & Hall, London, England, 1995; Watson, et al., Recombinant DNA, 2nd Ed., Scientific American Books, New York, New York, 1992 and Davis et al., Basic Methods in Molecular Biology, Elsevier Science Publishing Co., New York, New York, 1986, Alberts, et al., Molecular Biology of the Cell, 4th Ed., Garland Science, New York, New York, 2002.

### Example 2

### BLS-OMP31 Chimera Stability

The first proof that the BLS-OMP31 chimeric protein has adopted a decameric folding was obtained through the light scattering technique. This procedure was used to calculate the molecular size of the proteins in solution. To conduct these assays, a molecular exclusion column was joined to a light scattering detector. As the protein eluted from the column its molecular weight was determined.

According to this method, the calculated BLS molecular weight was 163 kDa while that of the BLS-OMP31 chimera was 215 kDa. The theoretical molecular weights of the native BLS and BLS-OMP31 decamers were of 174.4 and 197.8 kDa, respectively. Taking into account that this method is 90% accurate, this result suggests that the BLS-OMP31 chimera forms a decamer very much alike the native BLS protein.

The folding of chimeric proteins was studied through circular dichroism. The circular dichroism measurements were performed in the J-810 spectropolarimeter (Jasco, UK), set up at a reading speed of 100 nm/min, a response time of 4 s and a band width of 1 nm. Quartz trays of 1, 2 or 5 mm (Hellma) were used. The samples were assayed in a 50 mM pH 7 phosphate buffer. The overlay of the circular dichroism spectra of the BLS-OMP31 chimera and native BLS showed that the general folding of both proteins was very similar. *See* Figure 8. Thus, it was confirmed that the chimeric proteins folded properly like decamers, with a secondary structure comparable to that of the native protein.

Next, it was studied whether the substitutions at N-terminal of the chimeric proteins affected their stability. To this end, the induced denaturalization of the chimeras by guanidine hydrochloride (Gdm-HCl) and temperature was studied through circular dichroism.

From denaturalization-to-balance curves, it was possible to obtain several thermodynamic parameters, such as the change of free energy associated with unfolding. With this parameter, the conformational stability typical of a protein was evaluated. *See* Pace, et al, Methods Enzymol., 131: 266-80 (1986). To assess the change of free energy associated with the unfolding of each protein, the research data was adjusted to a theoretical descriptive curve. This diagram depicted the correlation of the equilibrium constant between the folded and unfolded stages of the chimeric protein and varying concentrations of the denaturalizing agent.

To conduct these experiments, the same amount of protein (0.1 µM of decamer) was incubated with increasing concentrations of the denaturalizing agent. The solutions for each point of the curve were prepared from a matrix solution of 6 M guanidine-HCl (ICN, ultra pure), 50 mM phosphate, pH 7. The samples were incubated for three hours at room temperature and were centrifuged before measurement. The circular dichroism measurements were performed in trays of 5 mm at 25 °C. The reversibility of the denaturalization by guanidine of the BLS native protein and the BLS-OMP31 chimeric protein was thus demonstrated.

The denaturalization-to-balance curves were adjusted to a two step dissociation model. According to this model, the decamer separated firstly into two equal pentamers. In the second step of the denaturalization, each of these pentamers dissociated further into five unfolded monomers. The formulas describing these transitions were derived from those already proposed for monomeric proteins. *See* Zylberman, et al, J. Biol. Chem., 279(9): 8093-8101 (2004).

The thermodynamic values obtained for the native BLS and BLS-OMP31 show that the replacement of the BLS N-terminal end did not affect the decamer stability. *See* Figure 9. Therefore, it was confirmed that the BLS chimeras form properly folded decamers and that their stability is similar to those of the native BLS.

### Example 3

### BLS-OMP31 Chimera Antigenicity and Immunogenicity

The antigenicity of the BLS-OMP31 was studied by measuring its capacity to bind to a specific monoclonal antibody against the inserted peptide (A59/10F09/G10 monoclonal antibody) and to a specific monoclonal antibody against the native BLS protein (BI24 monoclonal antibody). *See* Vizcaino, et al., Infect. Immun.. 69(11):7020-28, (2001); Cold baum, et al., J. Clin. Microbiol., 31:2141-2145 (1993). This procedure allows assaying the antigenicity of both the insert and the protein core. The antigenicity was assayed by ELISA. *See* Figure 10.

The BLS-OMP31 was identified both by the monoclonal antibody against the native BLS and by the monoclonal antibody against the inserted peptide. The native BLS was only identified by the first antibody, as expected. This result indicates that the inserted peptide preserved its antigenic properties when displayed by the chimera. In addition, it was also showed that the folding of the chimera did not affect the affinity of anti-BLS antibody to the protein core. Both antibodies detected up to 20 ng of chimera per well.

Then, the BLS-OMP31 capacity to induce a specific humoral immune response against the inserted peptide was evaluated. This capacity was analyzed in mice with and without the assistance of adjuvants. The experiment was performed with two groups of five mice each. The "AF" group received three doses, by intraperitoneal route, of 25 µg of protein in emulsion with a Freund's adjuvant at 0, 20 and 40-day intervals. The first dose was administered with a complete Freund's adjuvant. The remaining doses were administered with an incomplete Freund's adjuvant. The "PBS" group had the same treatment but the chimeric protein was injected without adjuvant. Blood was drawn 7 days after the third immunization and ' the sera reactivity against the OMP31 membrane protein was assayed by ELISA *See* Figure 11.

A strong response against OMP31 was obtained in the mice immunized with the chimera and the adjuvant. The serologic response obtained from the mice immunized with the chimeric protein with and without the adjuvant was also relevant. These results show that mice immunized with the BLS-OMP31 chimera with or without adjuvant have specific immune responses against the inserted peptide.

A rabbit was injected with BLS-OMP31 chimera with adjuvant according to the following protocol:
First dose, day 0: 200 µg BLS-OMP31 in 1 ml of PBS + 1 ml of complete Freund's adjuvant, by intramuscular injection.
Second dose, day 22: 200 µg BLS-OMP31 in 1 ml of PBS + 1 ml of incomplete Freund's adjuvant (IFA), by subcutaneous injection.
Third dose, day 45: 200 µg BLS-OMP31 in I ml of PBS + 1ml of IFA, by intramuscular injection.
Fourth dose, day 155: 200 µg BLS-OMP31 in 1 ml of PBS + 1ml of IFA, by subcutaneous injection.

Blood samples were drawn at 31^{st}, 52^{nd} and 180^{th} day. The samples were centrifuged and the serum was frozen.

The antisera collected after the 2^{nd}, 3^{rd} and 4^{th} doses of the antigen was titrated by ELISA against the OMP31 membrane protein. *See* Figure 12. The assayed antisera titer was of 3,200, 12,800 and 25,600 for sera corresponding to the 2^{nd}, 3^{rd} and 4^{th} doses, respectively. The base line was defined as the maximum dilution capable of identifying the antigen over the negative serum. The immunized rabbit showed a strong specific immune response against the OMP31. Since the anti native BLS serum did not identify the OMP31, this high reactivity was due to a response of specific antibodies against the peptide inserted in the chimera *See* Figure 13, negative control.

The OMP31 protein used in the ELISA assays was produced recombinantly in *E. coli.* Since this molecule is a membrane protein, it cannot be kept in an aqueous solution and was, therefore, obtained under denaturalizing conditions. In the assays performed, it was not demonstrated that the antisera were able to identify the OMP31 chimera in its native conformation as a bacterial membrane protein. This property is important to evaluate the potential effectivity of the chimera as an immunogen capable of providing humoral immunity against *Brucella.* To assess this property, ELISA assays were performed using a smooth and a rough strain of *B. melitensis H38,* as antigens. *See* Figure 13. The reactivity of a serum against whole bacteria is difficult to evaluate in general due to the complexity of the antigen used. However, the assay showed that the anti BLS-OMP31 serum specifically identified the OMP31 insert in the membrane of the rough strain. The reactivity of this serum against the smooth strain was not different from that shown by the anti native BLS serum This result is completely consistent with the data published for the A59/10F09/G10 monoclonal antibody. *See* Vizcaino, *et al., supra.* This antibody, specific for the insert included in the BLS-OMP31 chimera, has a strong reactivity with the rough, but not the smooth strain of *B. melitensis H38.*

### Example 4

### BLS-OMP31 DNA Vaccine

The codifying sequence for the BLS-Omp31 was subcloned in the vector pCI-neo (Promega, USA), including the restriction sites in the 5' ends (framed) of the primers and the Kozak consensus sequence (underlined). Hence, the following oligonucleotides were built:
**BLS-OMP31** "sense":
   5'TAAGAA **GAATCC ACCACCATG** CAT ACC GCC GGT TA 3'.
**BLS-OMP31** "anti-sense":
   5'TGT CCA CCA GTC AT **GCTAGCT** CAG ACA AGC GCG ATG C 3'.

Such sequence was amplified by PCR using the pET-BLS-OMP31 plasmid as a template. The PCR product and the vector were digested with the corresponding restriction enzymes and then a ligation reaction was performed. The obtained construct was checked by sequencing. The pCI-BLS-OMP31 plasmid was amplified in *E. coli* JM109 cells and further purified by "mega prep" columns (Quiagen, UK). DNA purity and concentration were assessed by spectrophotometry at 260/280 nm. The plasmid preparation contained less than 0.05 units of endotoxin per 100 µg of DNA, determined by a limulus amebocyte lysate analysis kit (Sigma, USA).

Groups of-Balb/c mice were inoculated with 100 µg of pCI-BLSOMP31 plasmid and the control plasmid without insert (pCI) in physiological solution by intramuscular (im) and intradermal (id) route at weeks 0, 2, 4 and 6. The animals blood was extracted by retroorbital route at days 0, 15, 30, 45, 60 and 75 after the first immunization. The sera were kept at -20°C for the detection of specific antibodies.

The anti-OMP31 humoral response induced by the immunization with the BLS-OMP31 DNA vaccine (pCI-BLSOMP31) was analyzed by indirect ELISA using the recombinant OMP31 protein as an antigen. After immunization, all animals developed a humoral immune response. A high level of the IgG isotype produced specifically against the Omp31 protein was observed. *See* Figure 14.

The preparation, amplification, purification and use of pCI-BLS-OMP31 plasmid as a DNA vaccine was performed using molecular biology techniques and methods for the preparation and administration of pharmaceutical compounds known in the art. *See* Schleef, M., Ed., Plasmids for Therapy and Vaccination, Wiley-VCH Verlag GmbH, Weinheim, Germany, 2001.

### Example 5

### Mixed BLS Chimeras

The fact that lumazine synthase of *Brucella spp.* dissociates reversibly when treated with high concentrations of guanidine chloride was used to construct mixed chimeras. *See* Zylberman, et al., J. Biol. Chem. 279(9):8093-8101 (2004). Two chimeras with marked differences in their insert size and isoelectric points were constructed. This strategy was followed in order to distinguish more easily the decamers formed by the mixed chimeras.

To this end, the KETcl peptide shown in Figure 15 was used in addition to the OMP31 peptide already described. The KETcl peptide derives from a protein of *Tenia solium* and has been described as highly protective against murine and pig neurocysticercosis. *See* Huerta, et al., Vaccine 20:62-266 (2001); Toledo, et al., Infect. Immun., 69:1766-1773 (2001).

The BLS-KETcl chimeric protein was obtained according to the following protocol:

### 1. Cloning

a) The pBLS-OMP31 plasmid was digested with the Nsi I and Afl II restriction enzymes to remove the codifying sequence corresponding to the 27 amino acids from the 48-74 sequence of the OMP31 protein. The OMP31 protein codifying sequence was extracted.
b) The codifying sequence for the 14 amino acids of the KETcl peptide was linked to the open cassette in a) by incubation overnight of the open cassette of the pBLS-OMP31^{-OMP31} plasmid with DNA T4 ligase enzyme at 16 °C. The BLS-KETcl reading frame was thus obtained. The insertion was confirmed by the sequencing of the reading frame. This reading frame was called BLS-KETcl chimera, of SEQ ID NO:10b, and the expression plasmid, pBLS-KETcl.

This procedure can also be performed following the protocol indicated in step 1) of Example 1 through the cleavage of BLSm cassette (SEQ ID NO:1b) with the Nsi and Afl I restriction enzymes and its further linkage with the KETcl insert. Thus, the BLS-KETcl reading frame and the pBLS-KETcl plasmid are also obtained.

This experience was repeated using the DNA sequences of the mutated BLS SEQ ID NO:2b, SEQ ID NO:3b, SEQ ID NO:4b, SEQ ID NO:5b, SEQ ID NO:6b and SEQ ID NO:7b. Similar results were obtained.

### 2. Transformation

A competent strain of *E*. *coli* BL21 (DE3) bacteria was transformed by thermal shock with the pBLS-KETcI plasmid obtained according to the protocol above. Afterwards, bacteria were cultured in agar plates including LB-agar/ampicilin to choose those transformed with the plasmid.

### 3. Expression and Purification of the BLS-KETcl Chimeric Protein

2 ml of a LB/ampicilin medium was inoculated to a colony extracted from agar plates for small-scale expression tests. The colony was shaked and incubated at 37 °C. The saturated culture was induced with 2 µl of 1M IPTG. Three hours later, 100 µl of culture was removed and centrifuged. The resulting pellet was resuspended in 25 µl of sample buffer 1X for its analysis by SDS-PAGE 17%:

A 5-ml preculture of the transformed strains was cultured to saturation according to the step above with 500 ml of LB/ampicilin. The culture was incubated and shaked at 37 °C. It was induced with 0.5 ml of 1M IPTG to reach an optical density of 0.6-0.8 at 600 nm. The culture was removed three hours later and was centrifuged at 4,000 g for 20 minutes. The pellet was resuspended in 15 ml of suspension solution (50 mM Tris, 5 mM EDTA, 1% Triton X-100, pH 8.0).

The suspension was sonicated for at 1 minute intervals every minute for 5 minutes and was centrifuged at 20,000 g for 30 minutes. The pellet was resuspended in 15 ml of suspension solution without Triton X-100 and the sonicate process was repeated. The procedure was repeated for a third time. The chimera expressed in the cytoplasm was contained in three sonicate supernatants while inclusion bodies were contained in the pellet.

The inclusion bodies were resuspended in PBS buffer with 8 M urea and were left overnight at room temperature. The resuspension was dialyzed against PBS for two days with a buffer change. The sample was centrifuged and the supernatant was dialyzed against buffer A (50mM Tris/HCl, pH 8.5). The first purification step was performed by anionic interchange chromatography in a MonoQ or a Q-Sepharose (Pharmacia, USA) column in a FPLC equipment (Pharmacia, USA). The sample was injected in the balanced column with buffer A and was eluted by linear gradient up to 50% of buffer B (buffer A + 1 M NaCl).

The purification second step was performed by chromatography in a Superdex 200 (Amersham, UK) molecular exclusion column. For this, the chimera peak was concentrated in a Centriprep (Millipore, USA) tube and injected in the column for elution with PBS. The presence of the chimeric protein in the peaks was evaluated by SDS-PAGE.

The construction of the BLS-KETcl chimeric gene was performed by using molecular biology techniques known in the art. *See* Sambrook, *et al., supra;* Brown, *supra;* Watson, *et al., supra;* Davis *et al., supra,* Alberts, *et al., supra.*

The BLS-OMP31, and BLS-KETcl chimeras were unfolded in 2 M of guanidine chloride, mixed in equimolar concentrations and re-associated through dialysis. Adding 2 M guanidine, the decamers were separated thus generated pentamers that preserved their secondary structure. When the guanidine was removed through dialysis, the pentamers were re-associated forming decamers again. *See* Zylberman, et al., J. Biol. Chem., 279(9): 8093-8101 (2004). In this manner, a mixture of BLS chimeras was produced. *See* Figure 16.

The re-association product was purified by interchange chromatography in a MonoQ (Amersham, UK) anionic interchange column. The results were compared to the elution profile of each separate chimera (a sample without dissociation) according to the following protocol: the BLS-KETcl chimera was eluted at 16.8% of buffer B (this particle was estimated the most basic in view of insert theoretical isoelectric point) and the BLS-OMP31 chimera was eluted at 35.8% of the same buffer. The re-associated sample was separated yielding three different peaks, the first corresponded to the pure BLS-KETcl chimera; the second and largest peak corresponded to the mixed chimera and the last peak corresponded to the pure BLS-OMP31 chimera, *See* Figure 17 A.

The sample corresponding to the second peak was analyzed by SDS-PAGE and native PAGE. The results demonstrated that the sample corresponded to a mixed chimera formed by KETcl peptide displayed in the five amino termini of one pentamer and by OMP31 peptide displayed in the five amino termini of the other pentamer. *See* Figures 17 B1 and B2.

This outcome showed that proposed strategy of separating, mixing and re-associating the modified proteins was effective to yield a mixed chimera where five copies of two different peptides were displayed by the BLS decameric structure. *See* Figure 16. The mixtures may have different characteristics depending on the nature of the inserts (e. g. one insert might be directed to an specific cell traffic while the other might induce a particular immune response).

### Example 6

### Immunization with Mixed BLS Chimeras

The mixture of the BLS-OMP31-KETcl chimeras was administered with adjuvants to mice to evaluate its capacity to induce a specific humoral immune response. The experiment was performed with a group of five mice. The group received three doses of 25 µg of protein in emulsion with a Freund's adjuvant by intraperitoneal route at 0, 20 and 40 days. The first dose was applied with a complete adjuvant. The second and third doses were applied with an incomplete adjuvant. Blood was drawn 7 days after the third immunization and the sera reactivity was assayed against the OMP31 and KETcl synthetic peptides by ELISA. *See* Figure 18.

A strong response against both peptides was obtained in immunized mice with the mixed chimeras with adjuvant. This demonstrated that mice immunized with the BLS-OMP31-KETcl mixed chimeras developed simultaneously a specific immune response against both inserts.

### Example 7

### Cellular Immune Response against BLS Chimeras

Groups of five BALB/c mice were immunized with 50 µg/mouse of the BLS-KETcl chimera emulsified in saponin and with 10 µg/mouse of the KETcl synthetic peptide emulsified in saponin to assess the specific cellular immune response induced by the BLS-KETcl chimera against the inserted peptide. The mice were immunized twice within a 10-day interval by subcutaneous route.

The spleens of both groups were aseptically removed three days after the last immunization. The spleen cells were resuspended in an RPMI 1640 Gibco (InVitrogen Corp., USA) culture medium, supplemented with L-glutamine (0.2mM), 2-mercaptoethanol (0.05mM), non-essential amino acids (0.01mM), penicillin (100 U/ml), streptomycin (100 µg/ml) and fetal bovine serum 10% (FBS). A culture medium, the KETcl peptide or the BLS-KETcl chimera (10 µg/ml) were added to different cell cultures. The cells were suspended in flat-bottom culture microplates at a concentration of 2 x 10⁵ cells per 200 µl of final volume. They were kept in a 5% CO₂ humidified environment at 37 °C.

After 72 hours, 1 µCi of [methyl-³H] timidine (Amersham Biosciences, UK) was added to each culture. The cells were seeded and the tritrated timidine level of incorporation was measured in a 1205 betaplate™ liquid scintillation counter (Wallac Oy, FI). All the assays were performed in triplicate.

The assay showed that the spleen cells of mice immunized with the BLS-KETcl chimera proliferated in cultures in the presence both of the peptide and the chimera. This result clearly indicated that the BLS-KETcl chimera was able to induce a specific cellular immune response against the KETcl peptide inserted in BLS. *See* Figure 19.

### Example 8

### Multidisplay of Protein Domains by BLS Chimeras

The BLS can be modified to display not only peptides but also complete protein domains in its ten amino termini. In order to demonstrate this alternate use a BLS-RBD3 chimera was constructed by linking the modified BLS codifying sequence and the codifying sequence of the RBD3 proteic domain of the murine protein Staufen-1. *See* Figures 20 and 21.

The BLS-KETcl chimeric protein was obtained according to the following protocol:

### 1. Cloning

a) The pBLS-OMP31 plasmid was digested with the Nsi I and Afl II restriction enzymes to remove the codifying sequence corresponding to the 27 amino acids from the 48-74 sequence of the OMP31 protein. The OMP31 protein codifying sequence was extracted.
b) The codifying sequence for the 75 amino acids of the RBD3 domain of the murine protein Staufen was linked to the open cassette in a) by incubation overnight of the open cassette of the pBLS-OMP31^{-OMP31} plasmid with DNA T4 ligase enzyme at 16 °C. The BLS-RBD3 reading frame was thus obtained The insertion was confirmed by the sequencing of the reading frame. This reading frame was called BLS-RBD3 chimera, of SEQ ID NO:11b, and the expression plasmid, pBLS-RBD3.

This procedure can also be performed following the protocol indicated in step 1) of Example 1 through the cleavage of BLSm cassette (SEQ ID NO:1b) with the Nsi and Afl I restriction enzymes and its further linkage with the RBD3 insert. Thus, the BLS-RBD3 reading frame and the pBLS-RBD3 plasmid are also obtained.

This experience was repeated using the DNA sequences of the mutated BLS SEQ ID NO:2b, SEQ ID NO:3b, SEQ ID NO:4b, SEQ ID NO:5b, SEQ ID NO:6b and SEQ ID NO:7b. Similar results were obtained.

### 2. Transformation

The pBLS-RBD3 plasmid obtained according to the above protocol was inserted in *E. coli* BL21 (DE3) bacteria by thermal shock transformation Afterwards, the bacteria were seeded in an LB medium in the presence of ampicillin. The gene expression was induced with IPTG 1 mM for 4 hours at 28 °C.

### 3. Expression and Purification of the BLS-RD3 Chimeric Protein

The protein was expressed in inclusion bodies, washed with 4 and 6 M urea solutions and solubilized in a buffer Tris/HCl 50 mM, urea 8 M, EDTA 5mM, β-ME 5 mM, PMSF 1 mM, pH 8 by magnetic stirring for 16 hours at 4 °C. The solubilized protein was purified under denaturalizing conditions in a Q-Sepharose ionic interchange column applying a linear gradient between 0 and 1 M NaCl in a buffer Tris/HCl 50 mM, 8 M urea, pH 8.5. The eluted protein was refolded by dialysis against the PBS buffer and purified by a heparin Hyper D column. For elution, a buffer Tris/HCl 50 mM, NaCl 1.2 M, pH 8 was used.

Figure 22 shows the SDS-PAGE, circular dichroism and light scattering analyses of the BLS-RBD3 chimera obtained through the method described above.

The SDS-PAGE analysis shows the high degree of purity of the BLS-RBD3 chimera obtained The small anomaly observed in electrophoretic mobility is attributed to the high density of the RBD3 domain positive charge. The similarity of the BLS-RBD3 circular dichroism spectrum with its theoretical spectrum, calculated by the combination of the spectra of the BLS and the RBD3 domain of the isolated murine protein Staufen-1, indicated that the chimera was well-folded. The chimera molecular weight (257 kDa), calculated from its run in the molecular filtering column connected in series to a light scattering detector and a refraction index detector, indicated that BLS-RBD3 chimera presented a decameric structure.

The construction of the BLS-RBD3 chimeric gene was performed by using molecular biology techniques known in the art. *See* Sambrook, *et al., supra;* Brown, *supra*; Watson, *et al., supra;* Davis *et al., supra,* Alberts, *et al., supra.*

### Example 9

### Production of Wide Spectrum Antibodies by Immunization with BLS Chimeras

The BLS-Staufen chimera was used as an immunogen to obtain antibodies against the Staufen RBD3 domain. 5 Balb/c mice were inoculated with 80 µg of the BLS-RBD3 chimera in a buffer 200 µl HCl 50mM, NaCl 1.2M, 50mM phosphate, pH 8 without adjuvant twice, at a 14-day interval by intraperitoneal route. As a control, 5 mice of the same strain were immunized with a mixture of BLS proteins and the Staufen RBD3 domain, in the same chimera-including mass. The mice were bleeded through retroorbital punction fifteen days after the last immunization. A serum was prepared through centrifugation at 1000 xg for 10 min. Sera reactivity against RBD3 was assayed by ELISA in 96-well plate with the glutathione S-transferase-RBD3 (GST-RBD3) fusion protein. Mouse anti-immunoglobulin conjugated to caprine peroxidase (DakoCytomation, USA) was used as a secondary antibody. The reaction was developed with orthophenildyamin (OPD) and was stopped with 4 N sulphuric acid. The optical density was determined by an ELISA reader at 492 nm.

Figure 23 shows a representative example of the humoral immune response developed by both groups. As observed, immunization with the BLS-RBD3 chimera caused a strong anti-RBD3 antibody response. No reactivity was observed against the peptide in mice inoculated with the BLS and RBD3 mixture.

### Example 10

### Production of Monocolonal Antibodies by Immunization with BLS Chimeras

The capacity to generate specific monoclonal antibodies against the OMP31 peptide was assessed from splenocytes of mice immunized with the BLS-OMP31 chimera The experiment was performed with a group of five mice. The group received three doses, by intraperitoneal route, of 25 µg of protein in emulsion to the medium with Freund's adjuvant at 0, 20 and 40-day intervals. At day 60, 25 µg of BLS-OMP31 dissolved in PBS was inoculated by peritoneal route in the mouse that showed a better response against the OMP31 peptide. The spleen of such mouse was removed and the splenocytes were merged with the NSO myeloma cells. The resulting hybridomas were selected in a HAT medium and their culture supernatants were assayed to measure reactivity against the OMP31 peptide by ELISA The hybridoma that showed a higher reactivity was cloned by limit dilution. Figure 24 shows the AcMo 37F7 reactivity against the OMP31 peptide and the whole OMP31 protein. A strong response against both was obtained. This demonstrated that mice immunized with the BLS-OMP31 chimera developed a specific immune response against the inserted peptide. This experience also shows that specific monoclonal antibodies could be produced using the BLS chimeras.

### Example 11

### Use of BLS Chimeras as Biosensors

Biosensors allow the detection of a specific interaction among macromolecules, which is visualized by the increase of a signal proportional to the mass accumulation on a reactive surface. The BLS-OMP31 modified protein was used to study the application of the BLS chimeras as a peptide and proteic domain carrier for the development of biosensors. To this end, the reaction between the BLS-OMP31 and the AcMo 37F7 described in the previous example was analyzed further.

The BLS-OMP31 chimera was used to derivatize a dextran carboximethyl plate (IAsys Affinity Sensors, Thermo, USA). For that purpose, a solution including 80 µg/ml of BLS-OMP31, in a buffer of 10 mM sodium acetate pH 5.5, was incubated in a plate previously derivatized by the EDC/NHS reagent. After immobilizing a signal corresponding to 800 arc sec (equal to 5 ng of antigen), the reactive surface was blocked with diethylamine. After derivatization, the anti-OMP31 AcMo 37F7 reactivity was studied, for which 50 µl of culture supernatant were incubated in the previously activated plate.

As observed in Figure 25, the AcMo 37F7 reacted strongly, providing a signal of approximately 300 arc sec. In the separation phase, the buffer PBS + Tween was washed and added, observing a drop in the signal corresponding to the AcMo separation of the solid phase. This example clearly shows that the chimera is able to detect antibodies directed against the peptide in the biosensor.

### Example 12

### Activation of Dendritic Cells by BLS

The BLS capacity of activating dendritic cells was analyzed. To that end, the activation levels of different markers were studied in these cells 18 hours after incubation with BLS. Bone marrow cells from Balb/c mice were cultured in Petri plates with a RPMI medium with 2mM L-glutamine, 100U/ml-penicillin, 100 µg/ml streptomycin, 50µM 2-mercaptoethanol and 10% fetal bovine serum (medium R10), supplemented with mouse granulocyte and macrophages colony stimulating factor (mGM-CSF) in an incubator with a 5% CO₂environment at 37 °C. The culture medium was replaced at days 3, 6 and 8. At day 9, the non-adhered cells were taken and centrifuged at 300 xg for 8 minutes. The cells were then incubated at a concentration of 2x10⁶ cells/ml in a final volume of 1 ml with BLS (1, 5 or 10µM) or without BLS in R10 medium for 18 hours (n=4). Afterwards, 4x10⁵ cells per 200µl of final volume were centrifuged and incubated with the following monoclonal antibodies (Pharmingen) conjugated to fluorescein isothiocyanate (FITC): anti-CD40, anti-CD80, anti I-A^{d} or anti-CD86, and with the anti-CD11c monoclonal antibody conjugated to phycoerythrin (PE). Three washings were performed and the cells were extracted with a FACScan cytometer (Becton Dickinson, USA). The obtained data was analyzed with the CellQuest (Becton Dickinson, USA) software.

In the cells incubated with BLS, within the subpopulation of CD11c⁺ (75-80%), significant increases were observed in the percentages and mean fluorescence of cells expressing CD40, CD80, I-A^{d} y CD86. The experiment was performed three times, obtaining similar results. Figure 26 shows representative histograms of the CD40 expression (A) and of the I-A^{d} (B) in CD11c⁺ cells treated with or without BLS.

A similar activation by BLS was observed in dendritic cells of C3H/HeJ mice (non responders to LPS) or when pre-incubating the BLS protein with polymyxin B, so as to eliminate LPS of *E*. *coli.*

These results demonstrated that the BLS is able to activate dendritic cells.

### Example 13

### Production of Molecular Assemblies with Protein Domains through Adaptor Peptides Linked to BLS Chimeras

The BLS protein could be modified in its amino termini to display whole proteic domains. This could be accomplished by the formation of molecular assemblies. In these clusters, complementary heterodimeric peptides are incorporated into the modified BLS protein and the target. Afterwards, the high affinity between the heterodimers links the target molecule and the modified BLS protein. The use of high affinity heterodimers is useful to avoid affecting the proper folding of the carrier protein. To demonstrate this application of the BLS chimeras, two heterodimerization peptides known in the art, RR₁₂EE₃45L y EE₁₂RR₃45L, were used. *See* Moll, et al., M. Protein Sci., 10:649-655 (2001). *See* Figure 27. This strategy allowed the construction of molecular assemblies, including ten copies of the domain combined with the BLS. The assembly was performed *in vitro,* which made possible to control the stoichiometry of the process. This system also enables expressing the antigen in a recombinant system different from BLS. *See* Figure 28.

### 1. Construction of Fusion Protein BLS-RR₁₂EE₃₄₅L

The peptide RR₁₂EE₃₄₅L was cloned in the N-terminal end of the BLS. *See* Figures 28 and 29. The K₄₉ residue located in BLS and RR₁₂EE₃₄₅L linker region was substituted for serine. The BLS-RR₁₂EE₃₄₅L chimeric gene was cloned in the pET11a vector. A competent strain of *E*. *coli* BL21 (DE3) bacteria was transformed with the resulting vector. Afterwards, the bacteria were cultured in a LB-agar/ampicilin culture medium. The gene expression was induced with 1M IPTG for 16 hours at 37 °C. The protein was expressed in the inclusion bodies.

The inclusion bodies were washed with a buffer 50 mM Tris/HCl. 5mM EDTA, 5 mM β-ME, 1 mM PMSF, pH 8. The dissolved protein was treated with a buffer 50 mM Tris/HCl, 8 M urea, 5mM EDTA, 5 mM β-ME, 1 mM PMSF, pH 8 and stirred magnetically for 16 hours at 4 °C. The resulting BLS-RR₁₂EE₃₄₅L chimera was purified under denaturalizing conditions by anionic interchange chromatography in a Q-Sepharose (Pharmacia, USA) column. The sample was eluted using a buffer 50 mM Tris/HCl, 8 M urea, pH 8.5 under a linear gradient of 0 to 1 M NaCl. The fusion protein was assayed by SDS-PAGE and light scattering analyses. *See* Figures 30 and 31.

The SDS-PAGE analysis showed that the fusion protein BLS-RR₁₂EE₃₄₅L had a high level of purity. The molecular weight of the protein, as determined by the light scattering technique, was 224 kDa. In addition, the protein showed a high-quality CD signal in the remote UV spectrum. These results suggest that the fusion protein is well folded and observes a decameric structure similar to the native BLS protein when in the presence of 8M urea The BLS structure is distorted at room temperature when the urea concentration is decreased. This is probably due to the binding of the RR₁₂EE₃₄₅L with itself

### 2. Construction of Peptide EE₁₂RR₃₄₅L

The peptide EE₁₂RR₃₄₅L was cloned in the C-terminal end of protein glutathione S-transferase (GST). This peptide is complementary to the RR₁₂EE₃₄₅L peptide displayed by the BLS fusion protein. *See* Figure 32.

The EE₁₂RR₃₄₅L peptide gene was cloned in the pGEX-4T1 vector. A competent strain of *E*. *coli* BL21 (DE3) bacteria was transformed with the resulting vector. Afterwards, the bacteria were cultured in a LB-agar/ampicilin culture medium at 37 °C. The gene expression was induced with 1mM IPTG for 16 hours at 37 °C. The bacteria were then sonicated.

The resulting EE₁₂RR₃₄₅L peptide was purified as a GST fusion protein using a glutathione/agarose matrix. The coupled complex was set in a column and was washed with a buffer 50 mM Tris, pH 8 until the peptide was absent from the eluent. Afterwards, the matrix was incubated with thrombine for 16 hours at room temperature to cleave the EE₁₂RR₃₄₅L from GST fusion protein. The peptide was then eluted with a buffer 50 mM Tris, pH 8 to purify it further. The resulting EE₁₂RR₃₄₅L peptide had a high level of purity.

### 3. Formation of Molecular Assembly between Fusion Protein BLS-RR₁₂EE₃₄₅L and Peptide EE₁₂RR₃₄₅L

One part of the fusion protein BLS-RR₁₂EE₃₄₅L was preincubated with 8 M urea, 50 mM Tris, 0.5 M NaCl, pH 8 for 15 minutes at 30°C. Four parts of the peptide EE₁₂RR₃₄₅L were preincubated in a buffer 50 mM Tris, 0.1 M NaCl, pH 8 for 15 minutes at 30°C. The fusion protein and peptide were mixed and incubated for 15 minutes at 30°C. The mixture remained soluble after incubation.

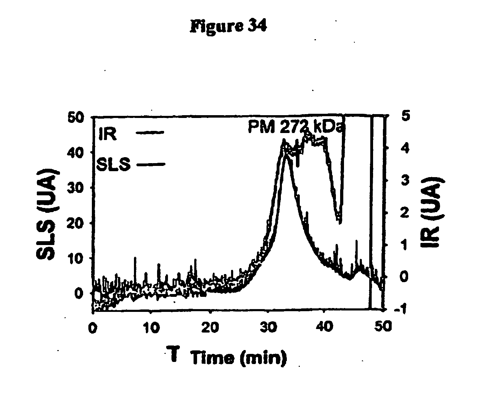

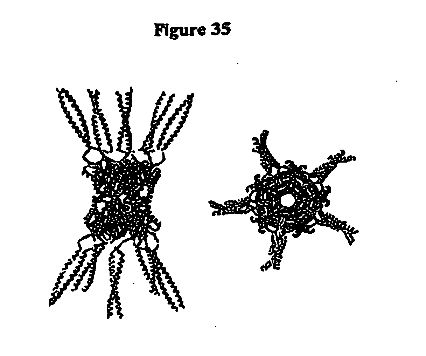

The resulting molecular assembly was assayed by light scattering analysis and its theoretical molecular weight was calculated (MW: 222.1 kDa). *See* Figure 34, This analysis showed that approximately 10 EE₁₂RR₃₄₅L peptides (MW: 5.6 kDa) coupled to the BLS-RR₁₂EE₃₄₅L decamer (MW: 222.1 kDa). *See* Figure 35.

In addition, the assembly showed a high-quality CD signal in the remote UV spectrum. These results suggest that molecular assemblies formed with modified BLS proteins using this technique are viable.

The present invention has been described in some detail and exemplified to facilitate its understanding and reproducibility.

### References

Arakawa, T., Yu, J., Chong, D., Hough, J., Engen, P. C. and Langridge, W. H. R. "A plant-based cholera toxin B subunit-insulin fusion protein protects against the development of autoimmune diabetes" Nature Biotech., 16:934-938, 1998.
Bacher A. and Fischer M. "Protein conjugates, methods, vectors, proteins and DNA for producing them, their use, and medicaments and vaccines containing a certain quantity of said protein conjugates" WO0053229.
Bachmann, M. F., Rohrer, U. H., Kundig, T. M., Burki, K., Hengartner, H. and Zinkernagel, R. M. "The influence of antigen organization on B cell responsiveness" Science, 262:1448-1451, 1993.
Baldi, P.C., Velikovsky, C., Braden, B.C., Giambartolomei, G.H., Fossati, C.A. and Goldbaum, F.A. "Structural, functional and immunological studies on a polymeric bacterial protein" Brazilian Journal of Medical and Biological Research, 33:741-747, 2000.
Baldi, P.C., Giambartolomei, G.H., Goldbaum, F.A., Abd6n, L.F., Velikovsky, C.A. Kittelberger, R. and Fossati, C.A. "Humoral immune response against LPS and cytoplasmic proteins of Brucella in cattle vaccinated with Brucella abortus S19 or experimentally infected with Yersinia enterocolitica 0:9" Clinical Diagnostic and Laboratory Immunology, 3 (4):472-476, 1996.
Braden, B.C., Velikovsky, C.A., Cauerhff, A., Polikarpov, I. and Goldbaum, F. A. "Divergence in macromolecular assembly: X-ray crystallographic structure analysis of lumazine synthase from Brucella abortus" Journal of Molecular Biology, 297:1031-1036, 2000.
Domingo, G.J., Orru, S. and Perham, R.N. "Multiple display of peptides and proteins on a macromolecular scaffold derived from a multienzyme complex" Journal of Molecular Biology, 305:259-267, 2001.
Domingo, G.J., Orru, S. and Perham, R.N. "Molecular display on multimeric protein scaffolds derived from the E2 component of the alphaketoacid dehydrogenase" WO0185208.
Goldbaum, F.A., Rubbi, C.P., Wallach, J.C., Miguel, S.E., Baldi, P.C. and Fossati, C.A "Differentiation between active and inactive human brucellosis by measuring antiprotein humoral immune responses" Journal of Clinical Microbiology, 30:604-607, 1992.
Goldbaum, F.A., Leoni, J., Wallach, J.C. and Fossati, C.A. "Characterization of an 18 kDa brucella cytoplasmic protein which appears to be a serological marker of active infection of both human and bovine brucellosis" Journal of Clinical Microbiology, 31:2141-2145, 1993.
Goldbaum, F.A., Polikarpov, I., Cauerhff, A., Velikovsky, C.A., Braden B.C. and Poljak, R.J. "Crystallization and preliminary X-ray analysis of the lumazine synthase from Brucella spp." Journal of Structural Biology, 123:175-178, 1998.
Goldbaum, F.A., Velikovsky, C.A., Baldi, P.C., Mörtl, S., Bacher, A. and Fossati, C.A. "The 18 kDa cytoplasmic protein of Brucella spp. is as enzyme with lumazine synthase activity" Journal of Medical Microbiology, 48:833-839, 1999.
Huerta, M., et al. "Synthetic peptide vaccine against Taenia solium pig cysticercosis: successful vaccination in a controlled field trial in rural Mexico" Vaccine, 20:262-266, 2001.
Laemmli, U.K. "Cleavage of structural proteins during the assembly of the head of bacteriophage T4." Nature, 227(259): 680-5, 1970.
Leclerc, C, and Ronco, J. "New approaches in vaccine development" Immunol. Today, 19(7):300-302, 1998.
Li, Y. , Mui, S., Brown, J. H. , Strand, J. , Reshetnikova, L. , Tobacman, L. S. y Cohen, C. 2002. The Crystal Structure of the C-Terminal Fragment of Striated-Muscle Alpha-Tropomyosin Reveals a Key Troponin T Recognition Site. Procedings Nacional Academy of Sciences USA, 99: 7378.
Moll, J., Ruvinov, S., Pastan, I. and Vinson, C. "Designed heterodimerizing leucine zippers with a ranger of pIs and stabilities up to 10-15 M" Protein Sci., 10:649-655, 2001.
Nieba, L. and Bachmann, M. F. "A new generation of vaccines" Moderns Aspects of Immunobiology, 1(2):36-39,2000.
Pace, C.N. "Determination and analysis of urea and guanidine hydrochloride denaturation curves" Methods Enzymol., 131: 266-80, 1986.
Redfield, N. New England Journal of Medicine, 316:673-678, 1998.
Renner, W.A., Bachmann, M., Hennecke, F., and Nieba, L. "Ordered molecular presentation of antigens, method of preparation and use" WO0032227.
Bachmann, M., Dunant N., Sebbel, P., Tissot, A., and Lechener, F. "Molecular antigen array" WO0185208.
Ritsert, K., Huber, R., Turk, D., Ladenstein, R., Schmidt-Base, K. and Bacher, A. "Studies on the lumazine synthase/riboflavin synthase complex of Bacillus subtilis: crystal structure analysis of reconstituted, icosahedral β-subunit capsids with bound substrate analogue inhibitor at 2.4 Å resolution" Journal of Molecular Biology, 253: 151-167,1995.
Toledo, A., et al. "Two epitopes shared by Taenia crassiceps and Taenia solium confer protection against murine T. crassiceps cysticercosis along with a prominent T1 response" Infect Immun 69:1766-1773, 2001.
Velikovsky, C. A., Cassataro, J., Sanchez, M., Fossati, C. A., Fainboim, L. and Spitz, M. "Single-shot plasmid DNA intrasplenic immunization for the production of monoclonal antibodies" Persistent Expression of DNA, J. Immunol. Meth., 244(1-2):1-7, 2000b.

### Sequences

SEQ ID NO:1a: 
MHSNQSCPLK TSFKIAFIQA RWHADIVDEA RKSFVAELAA KTGGSVEVEI FDVPGAYEIP 60
LHAKTLARTG RYAAIVGAAF VIDGGIYDHD FVATAVINGM MQVQLETEVP VLSVVLTPHH 120
FHESKEHHDF FHAHFKVKGV EAAHAALQIV SERSRIALV 159
SEQ ID NO:2a: 
MHSNQSCLKT SFKIAFIQAR WHADIVDEAR KSFVAELAAK TGGSVEVEIF DVPGAYEIPL 60
HAKTLARTGR YAAIVGAAFV IDGGIYDHDF VATAVINGMM QVQLETEVPV LSVVLTPHHF 120
HESKEHHDFF HAHFKVKGVE AAHAALQIVS ERSRIALV 158
SEQ ID NO:3a: 
MHSNQSLKTS FKIAFIQARW HADIVDEARK SFVAELAAKT GGSVEVEIFD VPGAYEIPLH 60
AKTLARTGRY AAIVGAAFVI DGGIYDHDFV ATAVINGMMQ VQLETEVPVL SVVLTPHHFH 120
ESKEHHDFFH AHFKVKGVEA AHAALQIVSE RSRIALV 157
SEQ ID NO:4a: 
MHSNQLKTSF KIAFIQARWH ADIVDEARKS FVAELAAKTG GSVEVEIFDV PGAYEIPLHA 60
KTLARTGRYA AIVGAAFVID GGIYDHDFVA TAVINGMMQV QLETEVPVLS VVLTPHHFHE 120
SKEHHDFFHA HFKVKGVEAA HAALQIVSER SRIALV 156
SEQ ID NO:5a: 
MHSNLKTSFK IAFIQARWHA DIVDEARKSF VAELAAKTGG SVEVEIFDVP GAYEIPLHAK 60
TLARTGRYAA IVGAAFVIDG GIYDHDFVAT AVINGMMQVQ LETEVPVLSV VLTPHHFHES 120
KEHHDFFHAH FKVKGVEAAH AALQIVSERS RIALV 155
SEQ ID NO:6a: 
MHSLKTSFKI AFIQARWHAD IVDEARKSFV AELAAKTGGS VEVEIFDVPG AYEIPLHAKT 60
LARTGRYAAI VGAAFVIDGG IYDHDFVATA VINGMMQVQL ETEVPVLSVV LTPHHFHESK 120
EHHDFFHAHF KVKGVEAAHA ALQIVSERSR IALV 154
SEQ ID NO:7a: 
MHLKTSFKIA FIQARWHADI VDEARKSFVA ELAAKTGGSV EVEIFDVPGA YEIPLHAKTL 60
ARTGRYAAIV GAAFVIDGGI YDHDFVATAV INGMMQVQLE TEVPVLSVVL TPHHFHESKE 120
HHDFFHAHFK VKGVEAAHAA LQIVSERSRI ALV 153
SEQ ID NO:8a: 
MHLEIRAAFL RQRNTALRTE VAELEQEVQR LENEVSQYET RYGPLGGGKL KTSFKIAFIQ 60
ARWHADIVDE ARKSFVAELA AKTGGSVEVE IFDVPGAYEI PLHAKTLART GRYAAIVGAA 120
FVIDGGIYDH DFVATAVING MMQVQLETEV PVLSVVLTPH HFHESKEHHD FFHAHFKVKG 180
VEAAHAALQI VSERSRIAL V 200
SEQ ID NO:9a: 
MHNAGYAGGK FKHPFSSFDK EDNEQVSGSL KTSFKIAFIQ ARWHADIVDE ARKSFVAELA 60
AKTGGSVEVE IFDVPGAYEI PLHAKTLART GRYAAIVGAA FVIDGGIYDH DFVATAVING 120
MMQVQLETEV PVLSVVLTPH HFHESKEHHD FFHAHFKVKG VEAAHAALQI VSERSRIALV 180
SEQ ID NO:10a: 
MHAPMSTPSA TSVRGSLKTS FKIAFIQARW HADIVDEARK SFVAELAAKT GGSVEVEIFD 60
VPGAYEIPLH AKTLARTGRY AAIVGAAFVI DGGIYDHDFV ATAVINGMMQ VQLETEVPVL 120
SVVLTPHHFH ESKEHHDFFH AHFKVKGVEA AHAALQIVSE RSRIALV 167
SEQ ID NO:11a: 
MHENLNKSEI SQVFEIALKR NLPVNFEVAR ESGPPHMKNF VTRVSVGEFV GEGEGKSKKI 60
SKKNAARAVL EQLRRLPLKT SFKIAFIQAR WHADIVDEAR KSFVAELAAK TGGSVEVEIF 120
DVPGAYEIPL HAKTLARTGR YAAIVGAAFV IDGGIYDHDF VATAVINGMM QVQLETEVPV 180
LSVVLTPHHF HESKEHHDFF HAHFKVKGVE AAHAALQIVS ERSRIALV 228
SEQ ID NO:1b: 
atgcatagca accaaagctg tccgcttaag acatccttta aaatcgcatt cattcaggcc 60
cgctggcacg ccgacatcgt tgacgaagcg cgcaaaagct ttgtcgccga actggccgca 120
aagacgggtg gcagcgtcga ggtagagata ttcgacgtgc cgggtgcata tgaaattccc 180
cttcacgcca agacattggc cagaaccggg cgctatgcag ccatcgtcgg tgcggccttc 240
gtgatcgacg gcggcatcta tcgtcatgat ttcgtggcga cggccgttat caacggcatg 300
atgcaggtgc agcttgaaac ggaagtgccg gtgctgagcg tcgtgctgac gccgcaccat 360
ttccatgaaa gcaaggagca tcacgacttc ttccatgctc atttcaaggt gaagggcgtg 420
gaagcggccc atgccgcctt gcagatcgtg agcgagcgca gccgcatcgc gcttgtctga 480
SEQ ID NO:2b: 
atgcatagca accaaagctg tcttaagaca tcctttaaaa tcgcattcat tcaggcccgc 60
tggcacgccg acatcgttga cgaagcgcgc aaaagctttg tcgccgaact ggccgcaaag 120
acgggtggca gcgtcgaggt agagatattc gacgtgccgg gtgcatatga aattcccctt 180
cacgccaaga cattggccag aaccgggcgc tatgcagcca tcgtcggtgc ggccttcgtg 240
atcgacggcg gcatctatcg tcatgatttc gtggcgacgg ccgttatcaa cggcatgatg 300
caggtgcagc ttgaaacgga agtgccggtg ctgagcgtcg tgctgacgcc gcaccatttc 360
catgaaagca aggagcatca cgacttcttc catgctcatt tcaaggtgaa gggcgtggaa 420
gcggcccatg ccgccttgca gatcgtgagc gagcgcagcc gcatcgcgct tgtctga 477
SEQ ID NO:3b: 
atgcatagca accaaagcct taagacatcc tttaaaatcg cattcattca ggcccgctgg 60
cacgccgaca tcgttgacga agcgcgcaaa agctttgtcg ccgaactggc cgcaaagacg 120
ggtggcagcg tcgaggtaga gatattcgac gtgccgggtg catatgaaat tccccttcac 180
gccaagacat tggccagaac cgggcgctat gcagccatcg tcggtgcggc cttcgtgatc 240
gacggcggca tctatcgtca tgatttcgtg gcgacggccg ttatcaacgg catgatgcag 300
gtgcagcttg aaacggaagt gccggtgctg agcgtcgtgc tgacgccgca ccatttccat 360
gaaagcaagg agcatcacga cttcttccat gctcatttca aggtgaaggg cgtggaagcg 420
gcccatgccg ccttgcagat cgtgagcgag cgcagccgca tcgcgcttgt ctga 474
SEQ ID NO:4b: 
atgcatagca accaacttaa gacatccttt aaaatcgcat tcattcaggc ccgctggcac 60
gccgacatcg ttgacgaagc gcgcaaaagc tttgtcgccg aactggccgc aaagacgggt 120
ggcagcgtcg aggtagagat attcgacgtg ccgggtgcat atgaaattcc ccttcacgcc 180
aagacattgg ccagaaccgg gcgctatgca gccatcgtcg gtgcggcctt cgtgatcgac 240
ggcggcatct atcgtcatga tttcgtggcg acggccgtta tcaacggcat gatgcaggtg 300
cagcttgaaa cggaagtgcc ggtgctgagc gtcgtgctga cgccgcacca tttccatgaa 360
agcaaggagc atcacgactt cttccatgct catttcaagg tgaagggcgt ggaagcggcc 420
catgccgcct tgcagatcgt gagcgagcgc agccgcatcg cgcttgtctg a 471
SEQ ID NO:5b: 
atgcatagca accttaagac atcctttaaa atcgcattca ttcaggcccg ctggcacgcc 60
gacatcgttg acgaagcgcg caaaagcttt gtcgccgaac tggccgcaaa gacgggtggc 120
agcgtcgagg tagagatatt cgacgtgccg ggtgcatatg aaattcccct tcacgccaag 180
acattggcca gaaccgggcg ctatgcagcc atcgtcggtg cggccttcgt gatcgacggc 240
ggcatctatc gtcatgattt cgtggcgacg gccgttatca acggcatgat gcaggtgcag 300
cttgaaacgg aagtgccggt gctgagcgtc gtgctgacgc cgcaccattt ccatgaaagc 360
aaggagcatc acgacttctt ccatgctcat ttcaaggtga agggcgtgga agcggcccat 420
gccgccttgc agatcgtgag cgagcgcagc cgcatcgcgc ttgtctga 468
SEQ ID NO:6b: 
atgcatagcc ttaagacatc ctttaaaatc gcattcattc aggcccgctg gcacgccgac 60
atcgttgacg aagcgcgcaa aagctttgtc gccgaactgg ccgcaaagac gggtggcagc 120
gtcgaggtag agatattcga cgtgccgggt gcatatgaaa ttccccttca cgccaagaca 180
ttggccagaa ccgggcgcta tgcagccatc gtcggtgcgg ccttcgtgat cgacggcggc 240
atctatcgtc atgatttcgt ggcgacggcc gttatcaacg gcatgatgca ggtgcagctt 300
gaaacggaag tgccggtgct gagcgtcgtg ctgacgccgc accatttcca tgaaagcaag 360
gagcatcacg acttcttcca tgctcatttc aaggtgaagg gcgtggaagc ggcccatgcc 420
gccttgcaga tcgtgagcga gcgcagccgc atcgcgcttg tctga 465
SEQ ID NO:7b: 
atgcatctta agacatcctt taaaatcgca ttcattcagg cccgctggca cgccgacatc 60
gttgacgaag cgcgcaaaag ctttgtcgcc gaactggccg caaagacggg tggcagcgtc 120
gaggtagaga tattcgacgt gccgggtgca tatgaaattc cccttcacgc caagacattg 180
gccagaaccg ggcgctatgc agccatcgtc ggtgcggcct tcgtgatcga cggcggcatc 240
 
tatcgtcatg atttcgtggc gacggccgtt atcaacggca tgatgcaggt gcagcttgaa 300
acggaagtgc cggtgctgag cgtcgtgctg acgccgcacc atttccatga aagcaaggag 360
catcacgact tcttccatgc tcatttcaag gtgaagggcg tggaagcggc ccatgccgcc 420
ttgcagatcg tgagcgagcg cagccgcatc gcgcttgtct ga 462
SEQ ID NO:8b: 
atgcatctgg aaatccgtgc ggcgttcctg cgtcagcgta acaccgcgct gcgtaccgaa 60
gttgcggaac tggaacagga agttcagcgt ctggaaaacg aagtttctca gtacgaaacc 120
cgttacggtc cgctgggtgg tggttctctt aagacatcct ttaaaatcgc attcattcag 180
gcccgctggc acgccgacat cgttgacgaa gcgcgcaaaa gctttgtcgc cgaactggcc 240
gcaaagacgg gtggcagcgt cgaggtagag atattcgacg tgccgggtgc atatgaaatt 300
ccccttcacg ccaagacatt ggccagaacc gggcgctatg cagccatcgt cggtgcggcc 360
ttcgtgatcg acggcggcat ctatcgtcat gatttcgtgg cgacggccgt tatcaacggc 420
atgatgcagg tgcagcttga aacggaagtg ccggtgctga gcgtcgtgct gacgccgcac 480
catttccatg aaagcaagga gcatcacgac ttcttccatg ctcatttcaa ggtgaagggc 540
gtggaagcgg cccatgccgc cttgcagatc gtgagcgagc gcagccgcat cgcgcttgtc 600
tga 603
SEQ ID NO:9b: 
atgcataacg ccggttacgc aggcggcaag ttcaagcatc cattttctag ctttgacaag 60
gaagacaacg aacaggtttc cggttcgctt aagacatcct ttaaaatcgc attcattcag 120
gcccgctggc acgccgacat cgttgacgaa gcgcgcaaaa gctttgtcgc cgaactggcc 180
gcaaagacgg gtggcagcgt cgaggtagag atattcgacg tgccgggtgc atatgaaatt 240
ccccttcacg ccaagacatt ggccagaacc gggcgctatg cagccatcgt cggtgcggcc 300
ttcgtgatcg acggcggcat ctatcgtcat gatttcgtgg cgacggccgt tatcaacggc 360
atgatgcagg tgcagcttga aacggaagtg ccggtgctga gcgtcgtgct gacgccgcac 420
catttccatg aaagcaagga gcatcacgac ttcttccatg ctcatttcaa ggtgaagggc 480
gtggaagcgg cccatgccgc cttgcagatc gtgagcgagc gcagccgcat cgcgcttgtc 540
tga 543
SEQ ID NO:10b: 
atgcatgccc cgatgagcac gccgagcgcc acgagcgtcc gcggtagcct taagacatcc 60
tttaaaatcg cattcattca ggcccgctgg cacgccgaca tcgttgacga agcgcgcaaa 120
agctttgtcg ccgaactggc cgcaaagacg ggtggcagcg tcgaggtaga gatattcgac 180
gtgccgggtg catatgaaat tccccttcac gccaagacat tggccagaac cgggcgctat 240
gcagccatcg tcggtgcggc cttcgtgatc gacggcggca tctatcgtca tgatttcgtg 300
gcgacggccg ttatcaacgg catgatgcag gtgcagcttg aaacggaagt gccggtgctg 360
agcgtcgtgc tgacgccgca ccatttccat gaaagcaagg agcatcacga cttcttccat 420
gctcatttca aggtgaaggg cgtggaagcg gcccatgccg ccttgcagat cgtgagcgag 480
cgcagccgca tcgcgcttgt ctga 504
SEQ ID NO:11b: 
atgcatgaaa acctcaataa atcggaaata agccaagtgt ttgaaattgc gctgaagcgg 60
aatttgcctg tgaattttga ggtggcccgg gagagtggcc caccacacat gaagaacttt 120
gtgaccaggg tttcagttgg ggaatttgta ggggaaggag aagggaaaag caagaagatc 180
tccaagaaga atgcggccag ggctgttctg gagcagctta ggaggctgcc acttaagaca 240
tcctttaaaa tcgcattcat tcaggcccgc tggcacgccg acatcgttga cgaagcgcgc 300
aaaagctttg tcgccgaact ggccgcaaag acgggtggca gcgtcgaggt agagatattc 360
gacgtgccgg gtgcatatga aattcccctt cacgccaaga cattggccag aaccgggcgc 420
tatgcagcca tcgtcggtgc ggccttcgtg atcgacggcg gcatctatcg tcatgatttc 480
gtggcgacgg ccgttatcaa cggcatgatg caggtgcagc ttgaaacgga agtgccggtg 540
ctgagcgtcg tgctgacgcc gcaccatttc catgaaagca aggagcatca cgacttcttc 600
catgctcatt tcaaggtgaa gggcgtggaa gcggcccatg ccgccttgca gatcgtgagc 660
gagcgcagcc gcatcgcgct tgtctga 687
SEQ ID NO:9c:

ttcttgaaga cgaaagggcc tcgtgatacg cctattttta taggttaatg tcatgataat 60
aatggtttct tagacgtcag gtggcacttt tcggggaaat gtgcgcggaa cccctatttg 120
tttatttttc taaatacatt caaatatgta tccgctcatg agacaataac cctgataaat 180
gcttcaataa tattgaaaaa ggaagagtat gagtattcaa catttccgtg tcgcccttat 240
tccctttttt gcggcatttt gccttcctgt ttttgctcac ccagaaacgc tggtgaaagt 300
aaaagatgct gaagatcagt tgggtgcacg agtgggttac atcgaactgg atctcaacag 360
cggtaagatc cttgagagtt ttcgccccga agaacgtttt ccaatgatga gcacttttaa 420
agttctgcta tgtggcgcgg tattatcccg tgttgacgcc gggcaagagc aactcggtcg 480
ccgcatacac tattctcaga atgacttggt tgagtactca ccagtcacag aaaagcatct 540
tacggatggc atgacagtaa gagaattatg cagtgctgcc ataaccatga gtgataacac 600

tgcggccaac ttacttctga caacgatcgg aggaccgaag gagctaaccg cttttttgca 660
caacatgggg gatcatgtaa ctcgccttga tcgttgggaa ccggagctga atgaagccat 720
accaaacgac gagcgtgaca ccacgatgcc tgcagcaatg gcaacaacgt tgcgcaaact 780
attaactggc gaactactta ctctagcttc ccggcaacaa ttaatagact ggatggaggc 840
ggataaagtt gcaggaccac ttctgcgctc ggcccttccg gctggctggt ttattgctga 900
taaatctgga gccggtgagc gtgggtctcg cggtatcatt gcagcactgg ggccagatgg 960
taagccctcc cgtatcgtag ttatctacac gacggggagt caggcaacta tggatgaacg 1020
aaatagacag atcgctgaga taggtgcctc actgattaag cattggtaac tgtcagacca 1080
agtttactca tatatacttt agattgattt aaaacttcat ttttaattta aaaggatcta 1140
ggtgaagatc ctttttgata atctcatgac caaaatccct taacgtgagt tttcgttcca 1200
ctgagcgtca gaccccgtag aaaagatcaa aggatcttct tgagatcctt tttttctgcg 1260
cgtaatctgc tgcttgcaaa caaaaaaacc accgctacca gcggtggttt gtttgccgga 1320
tcaagagcta ccaactcttt ttccgaaggt aactggcttc agcagagcgc agataccaaa 1380
tactgtcctt ctagtgtagc cgtagttagg ccaccacttc aagaactctg tagcaccgcc 1440
tacatacctc gctctgctaa tcctgttacc agtggctgct gccagtggcg ataagtcgtg 1500
tcttaccggg ttggactcaa gacgatagtt accggataag gcgcagcggt cgggctgaac 1560
ggggggttcg tgcacacagc ccagcttgga gcgaacgacc tacaccgaac tgagatacct 1620
acagcgtgag ctatgagaaa gcgccacgct tcccgaaggg agaaaggcgg acaggtatcc 1680
ggtaagcggc agggtcggaa caggagagcg cacgagggag cttccagggg gaaacgcctg 1740
gtatctttat agtcctgtcg ggtttcgcca cctctgactt gagcgtcgat ttttgtgatg 1800
ctcgtcaggg gggcggagcc tatggaaaaa cgccagcaac gcggcctttt tacggttcct 1860
ggccttttgc tggccttttg ctcacatgtt ctttcctgcg ttatcccctg attctgtgga 1920
taaccgtatt accgcctttg agtgagctga taccgctcgc cgcagccgaa cgaccgagcg 1980
cagcgagtca gtgagcgagg aagcggaaga gcgcctgatg cggtattttc tccttacgca 2040
tctgtgcggt atttcacacc gcatatatgg tgcactctca gtacaatctg ctctgatgcc 2100
gcatagttaa gccagtatac actccgctat cgctacgtga ctgggtcatg gctgcgcccc 2160
gacacccgcc aacacccgct gacgcgccct gacgggcttg tctgctcccg gcatccgctt 2220
acagacaagc tgtgaccgtc tccgggagct gcatgtgtca gaggttttca ccgtcatcac 2280
cgaaacgcgc gaggcagctg cggtaaagct catcagcgtg gtcgtgaagc gattcacaga 2340
tgtctgcctg ttcatccgcg tccagctcgt tgagtttctc cagaagcgtt aatgtctggc 2400
ttctgataaa gcgggccatg ttaagggcgg ttttttcctg tttggtcact gatgcctccg 2460
tgtaaggggg atttctgttc atgggggtaa tgataccgat gaaacgagag aggatgctca 2520
cgatacgggt tactgatgat gaacatgccc ggttactgga acgttgtgag ggtaaacaac 2580
tggcggtatg gatgcggcgg gaccagagaa aaatcactca gggtcaatgc cagcgcttcg 2640
ttaatacaga tgtaggtgtt ccacagggta gccagcagca tcctgcgatg cagatccgga 2700
acataatggt gcagggcgct gacttccgcg tttccagact ttacgaaaca cggaaaccga 2760
agaccattca tgttgttgct caggtcgcag acgttttgca gcagcagtcg cttcacgttc 2820
gctcgcgtat cggtgattca ttctgctaac cagtaaggca accccgccag cctagccggg 2880
tcctcaacga caggagcacg atcatgcgca cccgtggcca ggacccaacg ctgcccgaga 2940
tgcgccgcgt gcggctgctg gagatggcgg acgcgatgga tatgttctgc caagggttgg 3000
tttgcgcatt cacagttctc cgcaagaatt gattggctcc aattcttgga gtggtgaatc 3060
cgttagcgag gtgccgccgg cttccattca ggtcgaggtg gcccggctcc atgcaccgcg 3120
acgcaacgcg gggaggcaga caaggtatag ggcggcgcct acaatccatg ccaacccgtt 3180
ccatgtgctc gccgaggcgg cataaatcgc cgtgacgatc agcggtccag tgatcgaagt 3240
taggctggta agagccgcga gcgatccttg aagctgtccc tgatggtcgt catctacctg 3300
cctggacagc atggcctgca acgcgggcat cccgatgccg ccggaagcga gaagaatcat 3360
aatggggaag gccatccagc ctcgcgtcgc gaacgccagc aagacgtagc ccagcgcgtc 3420
ggccgccatg ccggcgataa tggcctgctt ctcgccgaaa cgtttggtgg cgggaccagt 3480
gacgaaggct tgagcgaggg cgtgcaagat tccgaatacc gcaagcgaca ggccgatcat 3540
cgtcgcgctc cagcgaaagc ggtcctcgcc gaaaatgacc cagagcgctg ccggcacctg 3600
tcctacgagt tgcatgataa agaagacagt cataagtgcg gcgacgatag tcatgccccg 3660
cgcccaccgg aaggagctga ctgggttgaa ggctctcaag ggcatcggtc gagatcccgg 3720
tgcctaatga gtgagctaac ttacattaat tgcgttgcgc tcactgcccg ctttccagtc 3780
gggaaacctg tcgtgccagc tgcattaatg aatcggccaa cgcgcgggga gaggcggttt 3840
gcgtattggg cgccagggtg gtttttcttt tcaccagtga gacgggcaac agctgattgc 3900
ccttcaccgc ctggccctga gagagttgca gcaagcggtc cacgctggtt tgccccagca 3960
ggcgaaaatc ctgtttgatg gtggttaacg gcgggatata acatgagctg tcttcggtat 4020
cgtcgtatcc cactaccgag atatccgcac caacgcgcag cccggactcg gtaatggcgc 4080
gcattgcgcc cagcgccatc tgatcgttgg caaccagcat cgcagtggga acgatgccct 4140
cattcagcat ttgcatggtt tgttgaaaac cggacatggc actccagtcg ccttcccgtt 4200
ccgctatcgg ctgaatttga ttgcgagtga gatatttatg ccagccagcc agacgcagac 4260
gcgccgagac agaacttaat gggcccgcta acagcgcgat ttgctggtga cccaatgcga 4320
ccagatgctc cacgcccagt cgcgtaccgt cttcatggga gaaaataata ctgttgatgg 4380
gtgtctggtc agagacatca agaaataacg ccggaacatt agtgcaggca gcttccacag 4440
caatggcatc ctggtcatcc agcggatagt taatgatcag cccactgacg cgttgcgcga 4500
gaagattgtg caccgccgct ttacaggctt cgacgccgct tcgttctacc atcgacacca 4560
ccacgctggc acccagttga tcggcgcgag atttaatcgc cgcgacaatt tgcgacggcg 4620
cgtgcagggc cagactggag gtggcaacgc caatcagcaa cgactgtttg cccgccagtt 4680

gttgtgccac gcggttggga atgtaattca gctccgccat cgccgcttcc actttttccc 4740
gcgttttcgc agaaacgtgg ctggcctggt tcaccacgcg ggaaacggtc tgataagaga 4800
caccggcata ctctgcgaca tcgtataacg ttactggttt cacattcacc accctgaatt 4860
gactctcttc cgggcgctat catgccatac cgcgaaaggt tttgcgccat tcgatggtgt 4920
ccgggatctc gacgctctcc cttatgcgac tcctgcatta ggaagcagcc cagtagtagg 4980
ttgaggccgt tgagcaccgc cgccgcaagg aatggtgcat gcaaggagat ggcgcccaac 5040
agtcccccgg ccacggggcc tgccaccata cccacgccga aacaagcgct catgagcccg 5100
aagtggcgag cccgatcttc cccatcggtg atgtcggcga tataggcgcc agcaaccgca 5160
cctgtggcgc cggtgatgcc ggccacgatg cgtccggcgt agaggatcga gatctcgatc 5220
ccgcgaaatt aatacgactc actatagggg aattgtgagc ggataacaat tcccctctag 5280
aaataatttt gtttaacttt aagaaggaga tatacatatg cataacgccg gttacgcagg 5340
cggcaagttc aagcatccat tttctagctt tgacaaggaa gacaacgaac aggtttccgg 5400
ttcgcttaag acatccttta aaatcgcatt cattcaggcc cgctggcacg ccgacatcgt 5460
tgacgaagcg cgcaaaagct ttgtcgccga actggccgca aagacgggtg gcagcgtcga 5520
ggtagagata ttcgacgtgc cgggtgcata tgaaattccc cttcacgcca agacattggc 5580
cagaaccggg cgctatgcag ccatcgtcgg tgcggccttc gtgatcgacg gcggcatcta 5640
tcgtcatgat ttcgtggcga cggccgttat caacggcatg atgcaggtgc agcttgaaac 5700
ggaagtgccg gtgctgagcg tcgtgctgac gccgcaccat ttccatgaaa gcaaggagca 5760
tcacgacttc ttccatgctc atttcaaggt gaagggcgtg gaagcggccc atgccgcctt 5820
gcagatcgtg agcgagcgca gccgcatcgc gcttgtctga gctagcatga ctggtggaca 5880
gcaaatgggt cgcggatccg gctgctaaca aagcccgaaa ggaagctgag ttggctgctg 5940
ccaccgctga gcaataacta gcataacccc ttggggcctc taaacgggtc ttgaggggtt 6000
ttttgctgaa aggaggaact atatccggat atcccgcaag aggcccggca gtaccggcat 6060
aaccaagcct atgcctacag catccagggt gacggtgccg aggatgacga tgagcgcatt 6120
gttagatttc atacacggtg cctgactgcg ttagcaattt aactgt 6166

## Claims

1. Isolated chimeric protein consisting of a peptide, a polypeptide, or a protein domain covalently linked to a modified lumazine synthase protein from *Brucella spp.,* wherein the first 8 N-termni amino acid residues have been deleted and the ninth Asn (N) has been replaced by Leu (L).

2. The isolated chimeric proteins, according to claim 1, wherein the amino acid sequence of the modified protein consists of SEQ ID NO:7a.

3. Isolated nucleotide sequences comprising the codifying sequences for the chimeric and modified proteins according to any one of claims 1 to 2, wherein the nucleotide sequences codifying for the first 8-N termini residues of the modified protein comprise restriction sites for enzymes Nsi I and Afl II.

4. The isolated DNA sequences, according to claim 3, consisting of the sequences selected from SEQ ID NO:1b, SEQ ID NO:2b, SEQ ID NO:3b, SEQ ID NO:4b, SEQ ID NO:5b, SEQ ID NO:6b, SEQ ID NO:7b, SEQ ID NO:9b, SEQ ID NO:10b, and SEQ ID NO:11b.

5. Vectors used as a vehicle comprising the nucleotide sequences according to any of claims 3 and 4.

6. The vectors, according to claim 5, wherein the vectors are viral or plasmidic.

7. Vectors according to claim 5, being the plasmid of SEQ ID NO: 9c.

8. The plasmid vectors according to claim 6, being the plasmid pBLS-OMP31, deposit number DSM 15546.

9. Cells transformed with the vectors according to any one of claims 5 to 8.

10. Cells transformed according to claim 9, wherein the cells are Eukaryotic or Prokaryotic cells.

11. Cells transformed according to claim 9, being Escherichia Coli cells.

12. The isolated chimeric proteins, according to claim 1, wherein the linked peptide, polypeptide or proteic domain is an antigen, toxin, agent or segment thereof, capable of inducing an immune response in an eukaryotic organism.

13. The isolated chimeric proteins according to claim 12, wherein the antigen is OMP-31, KET C1 or RBD3 or segment of OMP-31, KET C 1 or RBD3.

14. The isolated chimeric proteins, according to claim 13, having sequence SEQ ID NO:9a.

15. The isolated chimeric proteins, according to claim 13, having sequence SEQ ID NO:10a.

16. The isolated chimeric proteins, according to claim 13, having sequence SEQ ID NO:11a.

17. The isolated chimeric proteins, according to claim 12, wherein the immune response is humoral or cellular.

18. The isolated chimeric proteins, according to claim 12, wherein the eukaryotic organism is a mammalian specimen such as a murine, rabbit or human specimen.

19. The combination of two or more isolated chimeric proteins, according to claim 1, wherein the peptides, polypeptides or proteic domains linked to each modified protein is different.

20. The combination of two or more isolated chimeric proteins, according to claim 1, wherein the peptides, polypeptides or proteic domains linked to each modified protein is identical.

21. A pharmaceutical composition comprising at least one of the isolated chimeric proteins according to any one of claims 1 to 2.

22. A pharmaceutical composition, according to claim 21, comprising an acceptable pharmaceutical excipient.

23. A pharmaceutical composition, according to claim 22, wherein the acceptable pharmaceutical excipient is an adjuvant.

24. A pharmaceutical composition, according to claim 23 wherein the adjuvant consist of a Freund's adjuvant, a MDP dipeptide, saponin, tyrosine stearate, aluminum hydroxide, lymph cytokines, the native protein of lumazine synthase of Brucella spp or the modified proteins of lumazine synthase of Brucella spp.

25. A pharmaceutical composition comprising at least one of the isolated nucleotide sequences according to any one of claims 3-4.

26. A pharmaceutical composition, according to claim 25, comprising an acceptable pharmaceutical excipient.

27. A pharmaceutical composition, according to claim 26, wherein the acceptable pharmaceutical excipient is an adjuvant.

28. A pharmaceutical composition, according to claim 27 wherein the adjuvant, consist of a Freund's adjuvant, a MDP dipeptide, saponin, tyrosine stearate, aluminum hydroxide, lymph cytokines, the native protein of lumazine synthase of Brucella spp or the modified proteins of lumazine synthase of Brucella spp.

29. A pharmaceutical composition comprising at least one of the isolated chimeric proteins according to any of claims 1-2 and at least one of the isolated nucleotide sequences according to any of claims 3-4.

30. A pharmaceutical composition according to claims 23 or 27 for use in inducing an immune response in an eukaryotic organism.

31. A composition, according to claim 30, wherein the immune response is humoral or cellular.

32. A composition, according to claim 30, wherein the pharmaceutical composition is to be administered simultaneously or sequentially.

33. A composition, according to claim 30, wherein the eukaryotic organism is a mammalian specimen such as a murine, rabbit or human specimen.

34. A composition, according to claim 30, wherein the pharmaceutical composition is to be administered by subcutaneous, intravenous, intraperitoneal, intramuscular, oral or nasal routes or through a needle-free injection system.

35. A biosensor comprising: (a) a base where at least one isolated chimeric protein according to claim 1 is fixed and (b) said base is connected to measuring means which are able to determine if a ligand has attached to, or reacted with, the isolated chimeric protein.

36. A biosensor, according to claim 35, wherein the ligand is an antibody.

37. Protein conjugates comprising a ligand linked to the peptide, polypeptide or proteic domain connected to the modified proteins of lumazine synthase of Brucella spp. according to claim 1.

38. Protein conjugates, according to claim 37, wherein the link is a covalent bond, such as a peptidic bond.

39. Protein conjugates, according to claim 37, wherein the link is non-covalent.

40. Protein conjugates, according to claim 37, wherein the ligand and the chimeric protein are, linked through connecting sequences of a heterodimerization domain, such as sequences that codify for leucine zipper.

41. Isolated chimeric proteins, according to claim 1, wherein their quaternary structure is a decamer.

## Patentansprüche

1. Isoliertes chimäres Protein, bestehend aus einem Peptid, einem Polypeptid oder einer Proteindomäne, welches bzw. welche mit einem modifizierten Lumazinsynthaseprotein von Brucella spp. kovalent verbunden ist, wobei die ersten 8 N-terminalen Aminosäurereste deletiert wurden und das neunte Asn (N) durch Leu (L) ersetzt wurde.

2. Isolierte chimäre Proteine nach Anspruch 1, wobei die Aminosäuresequenz des modifizierten Proteins aus SED ID NO:7a besteht.

3. Isolierte Nukleotidsequenzen, umfassend die kodifizierenden Sequenzen für die chimären und modifizierten Proteine nach einem der Ansprüche 1 bis 2, wobei die Nukleotidsequenzen, die für die ersten 8 N-terminalen Reste des modifizierten Proteins kodifizieren, Restriktionsstellen für die Enzyme Nsi I und Afl II umfassen.

4. Isolierte DNA-Sequenzen nach Anspruch 3, bestehend aus den Sequenzen, die aus SEQ ID NO:1b, SEQ ID NO:2b, SEQ ID NO:3b, SEQ ID NO:4b, SEQ ID NO:5b, SEQ ID NO: 6b, SEQ ID NO:7b, SEQ ID NO:9b, SEQ ID NO:10b, SEQ ID NO:11b ausgewählt sind.

5. Vektoren, die als Vehikel verwendet werden, umfassend die Nukleotidsequenzen nach einem der Ansprüche 3 und 4.

6. Vektoren nach Anspruch 5, wobei die Vektoren viral oder plasmidisch sind.

7. Vektoren nach Anspruch 5, wobei es sich um das Plasmid von SEQ ID NO:9c handelt.

8. Plasmidvektoren nach Anspruch 6, wobei es sich um das Plasmid pBLS-OMP31 mit der Hinterlegungsnummer DSM 15546 handelt.

9. Zellen, die mit den Vektoren nach einem der Ansprüche 5 bis 8 transformiert sind.

10. Zellen, die nach Anspruch 9 transformiert sind, wobei die Zellen eukaryotische oder prokaryotische Zellen sind.

11. Zellen, die nach Anspruch 9 transformiert sind, wobei es sich um Escherichia coli-Zellen handelt.

12. Isolierte chimäre Proteine nach Anspruch 1, wobei das verbundene Peptid, Polypeptid oder die Proteindomäne ein Antigen, Toxin, Mittel oder Segment davon ist, das in der Lage ist, eine Immunreaktion in einem eukaryotischen Organismus hervorzurufen.

13. Isoliertes chimäres Protein nach Anspruch 12, wobei das Antigen OMP-31, KET C1 oder RBD3 oder ein Segment von OMP-31, KET C1 oder RBD3 ist.

14. Isolierte chimäre Proteine nach Anspruch 13 mit der Sequenz SEQ ID NO:9a.

15. Isolierte chimäre Proteine nach Anspruch 13 mit der Sequenz SEQ ID NO:10a.

16. Isolierte chimäre Proteine nach Anspruch 13 mit der Sequenz SEQ ID NO:11a.

17. Isolierte chimäre Proteine nach Anspruch 12, wobei die Immunreaktion humoral oder zellulär ist.

18. Isolierte chimäre Proteine nach Anspruch 12, wobei der eukaryotische Organismus ein Exemplar eines Säugetiers, wie beispielsweise ein Exemplar einer Maus, eines Kaninchens oder Menschen ist.

19. Kombination von zwei oder mehr isolierten chimären Proteinen nach Anspruch 1, wobei die Peptide, Polypeptide oder Proteindomänen, die mit jedem modifizierten Protein verbunden sind, unterschiedlich sind.

20. Kombination von zwei oder mehr isolierten chimären Proteinen nach Anspruch 1, wobei die Peptide, Polypeptide oder Proteindomänen, die mit jedem modifizierten Protein verbunden sind, identisch sind.

21. Pharmazeutische Zusammensetzung, umfassend mindestens eines der isolierten chimären Proteine nach einem der Ansprüche 1 bis 2.

22. Pharmazeutische Zusammensetzung nach Anspruch 21, umfassend einen annehmbaren pharmazeutischen Hilfsstoff.

23. Pharmazeutische Zusammensetzung nach Anspruch 22, wobei der annehmbare pharmazeutische Hilfsstoff ein Adjuvans ist.

24. Pharmazeutische Zusammensetzung nach Anspruch 23, wobei das Adjuvans aus einem Freundschen Adjuvans, einem MDP-Dipeptid, Saponin, Tyrosinstearat, Aluiniumhydroxid, Lymphzytokinen, dem nativen Protein von Lumazinsynthase von Brucella spp oder den modifizierten Proteinen von Lumazinsynthase von Brucella spp. besteht.

25. Pharmazeutische Zusammensetzung, umfassend mindestens eine der isolierten Nukleotidsequenzen nach einem der Ansprüche 3-4.

26. Pharmazeutische Zusammensetzung nach Anspruch 25, umfassend einen annehmbaren pharmazeutischen Hilfsstoff.

27. Pharmazeutische Zusammensetzung nach Anspruch 26, wobei der annehmbare pharmazeutische Hilfsstoff ein Adjuvans ist.

28. Pharmazeutische Zusammensetzung nach Anspruch 27, wobei das Adjuvans aus einem Freundschen Adjuvans, einem MDP-Dipeptid, Saponin, Tyrosinstearat, Aluminiumhydroxid, Lymphzytokinen, dem nativen Protein von Lumazinsynthase von Brucella spp oder den modifizierten Proteinen von Lumazinsynthase von Brucella spp besteht.

29. Pharmazeutische Zusammensetzung, umfassend mindestens eines der isolierten chimären Proteine nach einem der Ansprüche 1-2 und mindestens eines der isolierten Nukleotidsequenzen nach einem der Ansprüche 3-4.

30. Pharmazeutische Zusammensetzung nach Anspruch 23 oder 27 zur Verwendung beim Hervorrufen einer Immunreaktion in einem eukaryotischen Organismus.

31. Zusammensetzung nach Anspruch 30, wobei die Immunreaktion humoral oder zellulär ist.

32. Zusammensetzung nach Anspruch 30, wobei die pharmazeutische Zusammensetzung gleichzeitig oder nacheinander zu verabreichen ist.

33. Zusammensetzung nach Anspruch 30, wobei der eukaryotische Organismus ein Exemplar eines Säugetiers, wie beispielsweise ein Exemplar einer Maus, eines Kaninchens oder Menschen ist.

34. Zusammensetzung nach Anspruch 30, wobei die pharmazeutische Zusammensetzung durch subkutane, intravenöse, intraperitoneale, intramuskuläre, orale oder nasale Wege oder durch ein nadelfreies Injektionssystem zu verabreichen ist.

35. Biosensor umfassend: (a) eine Basis, an der mindestens ein isoliertes chimäres Protein nach Anspruch 1 fixiert ist, und (b) die Basis mit Messmitteln verbunden ist, die in der Lage sind festzustellen, wenn ein Ligand an das isolierte chimäre Protein angelagert ist oder mit diesem reagiert.

36. Biosensor nach Anspruch 35, wobei der Ligand ein Antikörper ist.

37. Proteinkonjugate, umfassend einen Liganden, der an das Peptid, Polypeptid oder die Proteindomäne gebunden ist, das bzw. die mit den modifizierten Proteinen von Lumazinsynthase von Brucella spp nach Anspruch 1 verbunden ist.

38. Proteinkonjugate nach Anspruch 37, wobei die Bindung eine kovalente Bindung, wie beispielsweise eine Peptidbindung, ist.

39. Proteinkonjugate nach Anspruch 37, wobei die Bindung nicht-kovalent ist.

40. Proteinkonjugate nach Anspruch 37, wobei der Ligand und das chimäre Protein durch verbindende Sequenzen einer Heterodimerisierungsdomäne, wie beispielsweise Sequenzen, die für Leucin-Zipper kodifizieren, gebunden sind.

41. Isolierte chimäre Proteine nach Anspruch 1, wobei ihr quaternärer Aufbau ein Dekamer ist.

## Revendications

1. Protéine chimère isolée constituée par un peptide, un polypeptide ou un domaine protéique lié de manière covalente à une protéine de lumazine synthase modifiée provenant de Brucella spp., dans laquelle les 8 premiers résidus d'acides aminés N-terminaux ont été supprimés et le neuvième Asn (N) a été remplacé par Leu (L).

2. Protéines chimères isolées, selon la revendication 1, dans lesquelles la séquence d'acides aminés de la protéine modifiée est constituée par SEQ ID N° : 7a.

3. Séquences nucléotidiques isolées comprenant les séquences codantes pour les protéines chimères et modifiées selon l'une quelconque des revendications 1 à 2, dans lesquelles les séquences nucléotidiques codant pour les 8 premiers résidus N-terminaux de la protéine modifiée comprennent des sites de restriction pour les enzymes Nsi I et Afl II.

4. Séquences d'ADN isolées, selon la revendication 3, constituées par les séquences sélectionnées parmi SEQ ID NO : 1b, SEQ ID NO : 2b, SEQ ID NO : 3b, SEQ ID NO : 4b, SEQ ID NO : 5b, SEQ ID NO : 6b, SEQ ID NO : 7b, SEQ ID NO : 9b, SEQ ID NO : 10b, SEQ ID NO : 11b.

5. Vecteurs utilisés en tant que véhicule comprenant les séquences nucléotidiques selon l'une quelconque des revendications 3 et 4.

6. Vecteurs, selon la revendication 5, dans lesquels les vecteurs sont viraux ou plasmidiques.

7. Vecteurs selon la revendication 5, qui sont le plasmide de SEQ ID NO : 9c.

8. Vecteurs plasmidiques selon la revendication 6, qui sont le plasmide pBLS-OMP31, numéro de dépôt DSM 15546.

9. Cellules transformées avec les vecteurs selon l'une quelconque des revendications 5 à 8.

10. Cellules transformées selon la revendication 9, dans lesquelles les cellules sont des cellules eucaryotes ou procaryotes.

11. Cellules transformées selon la revendication 9, qui sont des cellules d'Escherichia Coli.

12. Protéines chimères isolées, selon la revendication 1, dans lesquelles le peptide, le polypeptide ou le domaine protéique lié est un antigène, une toxine, un agent ou un segment de ceux-ci, capable d'induire une réponse immunitaire dans un organisme eucaryote.

13. Protéine chimère isolée selon la revendication 12, dans laquelle l'antigène est OMP-31, KET C1 ou RBD3 ou un segment d'OMP-31, de KET C1 ou de RBD3.

14. Protéines chimères isolées, selon la revendication 13, ayant la séquence SEQ ID NO : 9a.

15. Protéines chimères isolées, selon la revendication 13, ayant la séquence SEQ ID NO : 10a.

16. Protéines chimères isolées, selon la revendication 13, ayant la séquence SEQ ID NO : 11a.

17. Protéines chimères isolées, selon la revendication 12, dans lesquelles la réponse immunitaire est humorale ou cellulaire.

18. Protéines chimères isolées, selon la revendication 12, dans lesquelles l'organisme eucaryote est un spécimen mammifère tel qu'un spécimen murin, de lapin ou humain.

19. Combinaison de deux protéines chimères isolées ou plus, selon la revendication 1, dans lesquelles les peptides, polypeptides ou domaines protéiques liés à chaque protéine modifiée sont différents.

20. Combinaison de deux protéines chimères isolées ou plus, selon la revendication 1, dans lesquelles les peptides, polypeptides ou domaines protéiques liés à chaque protéine modifiée sont identiques.

21. Composition pharmaceutique comprenant au moins une des protéines chimères isolées selon l'une quelconque des revendications 1 à 2.

22. Composition pharmaceutique, selon la revendication 21, comprenant un excipient pharmaceutique acceptable.

23. Composition pharmaceutique, selon la revendication 22, dans laquelle l'excipient pharmaceutique acceptable est un adjuvant.

24. Composition pharmaceutique, selon la revendication 23, dans laquelle l'adjuvant est constitué par un adjuvant de Freund, un dipeptide MDP, la saponine, le stéarate de tyrosine, l'hydroxyde d'aluminium, les cytokines lymphatiques, la protéine native de la lumazine synthase de Brucella spp. ou les protéines modifiées de la lumazine synthase de Brucella spp.

25. Composition pharmaceutique comprenant au moins une des séquences nucléotidiques isolées selon l'une quelconque des revendications 3 à 4.

26. Composition pharmaceutique, selon la revendication 25, comprenant un excipient pharmaceutique acceptable.

27. Composition pharmaceutique, selon la revendication 26, dans laquelle l'excipient pharmaceutique acceptable est un adjuvant.

28. Composition pharmaceutique, selon la revendication 27, dans laquelle l'adjuvant est constitué par un adjuvant de Freund, un dipeptide MDP, la saponine, le stéarate de tyrosine, l'hydroxyde d'aluminium, les cytokines lymphatiques, la protéine native de la lumazine synthase de Brucella spp. ou les protéines modifiées de la lumazine synthase de Brucella spp.

29. Composition pharmaceutique comprenant au moins une des protéines chimères isolées selon l'une quelconque des revendications 1 à 2 et au moins une des séquences nucléotidiques isolées selon l'une quelconque des revendications 3 à 4.

30. Composition pharmaceutique selon les revendications 23 ou 27 destinée à être utilisée dans l'induction d'une réponse immunitaire dans un organisme eucaryote.

31. Composition, selon la revendication 30, dans laquelle la réponse immunitaire est humorale ou cellulaire.

32. Composition, selon la revendication 30, dans laquelle la composition pharmaceutique est à administrer simultanément ou séquentiellement.

33. Composition, selon la revendication 30, dans laquelle l'organisme eucaryote est un spécimen mammifère tel qu'un spécimen murin, de lapin ou humain.

34. Composition, selon la revendication 30, dans laquelle la composition pharmaceutique est à administrer par les voies sous-cutanée, intraveineuse, intrapéritonéale, intramusculaire, orale ou nasale ou à travers un système d'injection sans aiguille.

35. Biocapteur comprenant : (a) une base où au moins une protéine chimère isolée selon la revendication 1 est fixée et (b) ladite base est reliée à des moyens de mesure qui sont capables de déterminer si un ligand s'est lié à, ou a réagi avec, la protéine chimère isolée.

36. Biocapteur, selon la revendication 35, dans lequel le ligand est un anticorps.

37. Conjugués protéiques comprenant un ligand lié au peptide, au polypeptide ou au domaine protéique relié aux protéines modifiées de lumazine synthase de Brucella spp. selon la revendication 1.

38. Conjugués protéiques, selon la revendication 37, dans lesquels la liaison est une liaison covalente, telle qu'une liaison peptidique.

39. Conjugués protéiques, selon la revendication 37, dans lesquels la liaison est une liaison non covalente.

40. Conjugués protéiques, selon la revendication 37, dans lesquels le ligand et la protéine chimère sont liés par le biais de séquences de liaison d'un domaine d'hétérodimérisation, telles que des séquences qui codent pour un domaine leucine zipper.

41. Protéines chimères isolées, selon la revendication 1, dans lesquelles leur structure quaternaire est un décamère.
